(19) Europäisches Patentamt
European Patent Office
Office européen des brevets

(11) EP 4 294 424 B1

(12) EUROPEAN PATENT SPECIFICATION

(45) Date of publication and mention of the grant of the patent:
**11.02.2026 Bulletin 2026/07**

(21) Application number: **22707673.4**

(22) Date of filing: **17.02.2022**

(51) International Patent Classification (IPC):
***A61K 38/26** (2006.01)* ***A61P 1/00** (2006.01)*

(52) Cooperative Patent Classification (CPC):
**A61K 38/26; A61P 1/00**

(86) International application number:
**PCT/EP2022/054001**

(87) International publication number:
**WO 2022/175410 (25.08.2022 Gazette 2022/34)**

# (54) GLP-1/GLP-2 DUAL AGONIST FOR TREATING SHORT BOWEL SYNDROME

GLP-1/GLP-2-DUALAGONIST ZUR BEHANDLUNG DES KURZDARMSYNDROMS

DOUBLE AGONISTE DU GLP-1/GLP-2 POUR LE TRAITEMENT DU SYNDROME DU GRÊLE COURT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2021 EP 21158004**

(43) Date of publication of application:
**27.12.2023 Bulletin 2023/52**

(73) Proprietor: **Zealand Pharma A/S**
**2860 Søborg (DK)**

(72) Inventors:
- **GRIFFIN, Jonathan**
  **2860 Søborg (DK)**
- **ERIKSSON, Per-Olof**
  **2860 Søborg (DK)**
- **RUSSELL, Wayne, Shaun**
  **2860 Søborg (DK)**
- **BERNER-HANSEN, Mark**
  **2860 Søborg (DK)**

(74) Representative: **D Young & Co LLP**
**3 Noble Street**
**London EC2V 7BQ (GB)**

(56) References cited:
**WO-A1-2018/103868** **WO-A1-2018/104558**
**WO-A1-2018/104560** **WO-A1-2018/104561**

- **J. SKARBALIENE ET AL: "PT01.2: ZP7570: A Novel GLP-1/GLP-2 Dual Acting Peptide with Potential as the Next Generation Therapy for Short Bowel Syndrome", CLINICAL NUTRITION, vol. 38, 1 September 2019 (2019-09-01), GB, pages S33, XP055734521, ISSN: 0261-5614, DOI: 10.1016/S0261-5614(19)32542-7**
- **K.B. MADSEN ET AL: "Acute effects of continuous infusions of glucagon-like peptide (GLP)-1, GLP-2 and the combination (GLP-1+GLP-2) on intestinal absorption in short bowel syndrome (SBS) patients. A placebo-controlled study", REGULATORY PEPTIDES, vol. 184, 1 June 2013 (2013-06-01), pages 30 - 39, XP055183143, ISSN: 0167-0115, DOI: 10.1016/j.regpep.2013.03.025**
- **MASSIRONI SARA ET AL: "Understanding short bowel syndrome: Current status and future perspectives", DIGESTIVE AND LIVER DISEASE, W.B. SAUNDERS, GB, vol. 52, no. 3, 28 December 2019 (2019-12-28), pages 253 - 261, XP086063901, ISSN: 1590-8658, [retrieved on 20191228], DOI: 10.1016/J.DLD.2019.11.013**
- **J. SKARBALIENE ET AL: "PT01.2: ZP7570: A Novel GLP-1/GLP-2 Dual Acting Peptide with Potential as the Next Generation Therapy for Short Bowel Syndrome", 14 August 2019 (2019-08-14), XP055734520, Retrieved from the Internet <URL:https://doi.org/10.1016/S0261-5614(19)32542-7> [retrieved on 20200928], DOI: 10.1016/S0261-5614(19)32542-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The complete document including Reference Table(s) and the Sequence Listing(s) can be downloaded from the EPO website

## Description

### Field of the Invention

The present invention relates to a composition comprising a GLP-1/GLP-2 dual agonist for use in a method for the treatment and/or prevention of diarrhea in a patient having undergone surgical resection of the bowel.

### Background to the Invention

Bowel resections are performed to treat and prevent diseases and conditions that affects or relates to the bowel. There are various clinical situations in which a bowel resection may be advisable, for example to treat cancer in the small intestine, colon, rectum or anus, to treat or relieve symptoms of cancer that has spread to the intestine, to remove a blockage in the intestine (called a bowel obstruction), to remove as much cancer as possible (called debulking), to remove precancerous conditions before they become cancer (called prophylactic surgery), to remove parts of the colon that are damaged by an inflammatory bowel disease (for example Crohn’s disease) or diverticulitis, or to manage a bleeding source, stricture/stenosis, tear or perforation. For example, colorectal cancer was the fourth most commonly diagnosed cancer in the United States in 2013. According to the 2010-2012 National Cancer Institute cancer fact sheet, approximately 4.5% of the US population will be diagnosed with colorectal cancer at some point during their lifetime. Surgery is the most common treatment for resectable colorectal cancer. The GLP-2 analogue teduglutide is approved for the treatment of SBS patients who are stable following a period of intestinal adaptation after surgery. Teduglutide is shown to have some effect on diarrhea (Jeppesen 2012) and an intermittent effect on treatment of diarrhea in SBS mice (Reiner *et al.* 2020). Diarrhea is loose, watery stools where defecation appears more than three times a day. For patients having SBS, diarrhea may be chronic, meaning that the diarrhea persist for more than 2 weeks.

However, there is still a need for an effective long-term management of diarrhea in patients who have undergone surgical resection of the bowel.

J. Skarbaliene et al. "PT01.2: ZP7570: A Novel GLP-1/GLP-2 Dual Agonist Peptide with Potential as the Next Generation Therapy for Short Bowel Syndrome" CLINICAL NUTRITION, vol. 38, 1 September 2019 relates to a GLP-1/GLP-2 agonist peptide for treating SBS.WO2018/104561 A1, WO2018/104560 A1 and WO2018/104558 A1 relate to compounds having agonist activity at the GLP-1 and GLP-2 receptors.

WO2018/103868 A1 relates to acylated GLP-1/GLP-2 dual agonists which may be used in the prophylaxis or treatment of intestinal damage and dysfunction, regulation of body weight and prophylaxis or treatment of metabolic dysfunction.

### Summary of the Invention

The present inventors have surprisingly found that administering a composition comprising a GLP-1/GLP-2 dual agonist to a patient can be used for the treatment and/or prevention of diarrhea in patients that have undergone surgical resection of the bowel.

The present invention is defined by the appended claims and it relates to a composition comprising a GLP-1/GLP-2 dual agonist which is Hy-H[Aib]EGSFTSELATILD[K([17-carboxy-heptadecanoyl]-isoGlu)]QAARDFIAWLIQHKITD-OH (Compound 18) or a pharmaceutically acceptable salt or solvate thereof, for use in a method for the treatment and/or prevention of diarrhea in a patient having undergone surgical resection of the bowel.

### Figure legends

**Figure 1:** Anatomy of bowel and illustration of resection. In wild-type mice either a 40% ileum plus cecum resection (ICR) for animals then allocated to the vehicle or compound 18 (Cpd 18) treated groups was carried out, or for the sham group an anastomosis was performed.

**Figure 2:** The initial weight of the animals for sham (n=9), Vehicle (n=12) and Cpd 18 (n=15).

**Figure 3:** The initial age of the animals for sham (n=9), Vehicle (n=12) and Cpd 18 (n=16).

**Figure 4:** The resection length in cm for sham (n=9), Vehicle (n=12) and Cpd 18 (n=16).

**Figure 5:** Postoperative survival of animals for sham (n=11), Vehicle (n=24) and Cpd 18 (n=26).

**Figure 6:** Postoperative body weight of animals (relative to initial weight) for sham (n=9), Vehicle (n=12) and Cpd 18 (n=14).

**Figure 7:** Postoperative dietary intake for sham (n=9), Vehicle (n=12) and Cpd 18 (n=14).

**Figure 8:** Postoperative stool water content for sham (n=9, circle), Vehicle (n=12, triangles) and Cpd 18 (n=14, squares).

**Figure 9:** Picture of postoperative stool consistency at day 5 (top), day 7 (middle) and day 14 (bottom) for vehicle (V), Compound 18 (Cpd 18) and Sham.

**Figure 10:** The Bristol Stool Score is a diagnostic medical tool designed to classify the form of human feces into seven categories. It is used in both clinical and experimental fields.

## Detailed Description of the Invention

The present inventors have surprisingly found that administering a composition comprising a GLP-1/GLP-2 dual agonist to a patient can be used for the treatment and/or prevention of diarrhea, loose stools, urgency, incontinence, high fecal output and/or high stomal output in patients that have undergone surgical resection of the bowel. The inventors have found that said composition improves and/or normalises defecation.

### Defecation Conditions

Patients having undergone surgical resection of the bowel suffer from a number of conditions and/or disorders which are all related to amount of water in the feces and/or the fecal output. The patients may suffer from diarrhea, loose stools, urgency, incontinence, high fecal output and/or high stomal output.

The invention provides a composition comprising a GLP-1/GLP-2 dual agonist for use in a method for the treatment and/or prevention of diarrhea in a patient having undergone surgical resection of the bowel.

The term "diarrhea" means the condition of having at least three loose, liquid, or watery defecations per day. "Diarrhea" corresponds to stools of Type 6 or Type 7 on the Bristol scale, as shown in Figure 10. The condition includes having loose stool or watery stool.

By the term "loose stool" is meant that the stool is watery, soft/mushy and are shapeless. The water content in loose stool is higher than in normal stool (stool rating 4 on the Bristol Stool Scale).

The term "urgency" or "fecal urgency" describes the experience by the patient that defecation is immediate, and the patient urgently needs to visit the toilet.

"Incontinence" or "fecal incontinence" describes the inability to control bowel movements, causing stool to leak unexpectedly from the rectum. Fecal incontinence ranges from an occasional leakage of stool while passing gas to a complete loss of bowel control.

The term "fecal output" means the amount of feces. The terms "feces" and "stool" are used interchangeably.

The present invention relates to a composition comprising a GLP-1/GLP-2 dual agonist for use in a method for the treatment and/or prevention of diarrhea in a patient having undergone surgical resection of the bowel. For patients having undergone bowel resection, their ability to take up nutrients and water from the food may be impaired. Such patients may suffer from a number of conditions and/or disorders which are related to having a high amount of water in the feces, e.g. leading to conditions like diarrhea, loose stools, urgency, incontinence, high fecal output and/or high stomal output. The conditions or disorders may be acute or may be chronic.

In one aspect of the invention, about 0.5, 0.6, 0.7, 0.8, 0.9, 1.0, 2.0, 3.0, 4.0, 5.0, 6.0, 7.0, 8.0, 9.0, 10.0, 11.0, 12.0, 13.0, 14.0, 15.0, 16.0, 17.0, 18.0, 19.0, or 20.0% or more, such as about 30, 40, 50, 60, 70, 80 or 90% of the bowel of said patient is removed.

Said patient may have short bowel syndrome secondary to one or more of digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, ulcerative colitis, small intestine damage Crohn’s disease, mesenteric infarction, volvulus, multiple strictures due to adhesions or radiation, vascular ischemia, necrotising enteral colitis (NEC), intestinal malformations, intestinal atresia, bowel cancer, surgical complications, acute injury, for example a stab injury.

In one aspect said patient has short bowel syndrome. Some patients having undergone surgical resection of the bowel may have a stoma such as a colostomy or an ileostomy.

The present invention relates to a composition comprising a GLP-1/GLP-2 dual agonist for use in a method for the treatment and/or prevention of diarrhea in a patient having undergone surgical resection of the bowel, wherein said

treatment and/or prevention reduces the frequency of daily defecations. In one embodiment of the invention, the frequency of daily defecations is reduced by at least 30%, such as at least 40%, at least 50% or at least 60%.

The present invention relates to a composition comprising a GLP-1/GLP-2 dual agonist for use in a method for the treatment and/or prevention of diarrhea in a patient having undergone surgical resection of the bowel, wherein said treatment and/or prevention reduces the number of daily defecations by at least one defecation. In one embodiment of the invention, the number of daily defecations is reduced by at least two defecations, such as at least three defecations, at least four defecations, at least six defecations, at least seven defecations, at least eight defecation, at least nine defecations or at least 10 defecations. In one embodiment, the number of daily defecations is reduced to one to five daily defecations, such as reduced to one to four daily defecations, reduced to one to three daily defecations or reduced to one to two daily defecations.

In an aspect of the invention, the use of a composition comprising a GLP-1/GLP-2 dual agonist in a method for the treatment and/or prevention of diarrhea in a patient having undergone surgical resection of the bowel, the treatment improves the consistency of the feces to a consistency comparable to the consistency (the thickness and/or viscosity) of feces from a human not suffering from said conditions. The treatment and/or prevention reduces the amount of water in the feces of said patient to an amount comparable to the amount of water in normal feces (feces rated 4 on the Bristol Stool Score).

In one aspect of the invention, the treatment reduces the amount of water in the feces, allowing the Bristol Stool Score of the feces to be reduced by at least 1, e.g. scoring 7 before treatment and 6 or lower when treated. In one embodiment, the amount of water in feces is reduced to allow a Bristol score reduction of at least 2, such as at least 3.

The Bristol stool scale is a diagnostic medical tool designed to classify the form of human feces into seven categories. It is used in both clinical and experimental fields. The scale is shown in Figure 10 and defines seven types of stools:

1. Separate hard lumps, like nuts (difficult to pass and can be black)
2. Sausage-shaped, but lumpy
3. Like a sausage but with cracks on its surface (can be black)
4. Like a sausage or snake, smooth and soft (average stool, normal stool)
5. Soft blobs with clear cut edges
6. Fluffy pieces with ragged edges, a mushy stool (diarrhea)
7. Watery, no solid pieces, entirely liquid (diarrhea)

In one aspect the invention provides a composition comprising a GLP-1/GLP-2 dual agonist for use in the method for the treatment and/or prevention of diarrhea in a patient having undergone surgical resection of the bowel, wherein the treatment reduces the amount of water in the feces of said patient by at least 20% compared to the amount of water in the feces of said patient before starting the treatment with said composition. In one embodiment of the invention, the amount of water in the feces may be reduced by at least 25%, such as at least 30%, at least 35%, at least 40%, at least 45%, at least 50%, at least 60% or at least 70% compared to the amount of water in the feces of said patient before starting the treatment with said composition.

In one aspect the invention provides a composition comprising a GLP-1/GLP-2 dual agonist for use in the method for the treatment and/or prevention of diarrhea in a patient having undergone surgical resection of the bowel, wherein the treatment reduces the fecal or stomal output of said patient by at least 30% compared to the fecal or stomal output of said patient before starting the treatment with said composition. In one embodiment of the invention, the amount of water in the feces may be reduced by at least 35%, at least 40%, at least 45%, at least 50%, at least 60% or at least 70% compared to the amount of water in the feces of said patient before starting the treatment with said composition.

In one aspect the invention provides a composition comprising a GLP-1/GLP-2 dual agonist for use in the method for the treatment and/or prevention of diarrhea in a patient having undergone surgical resection of the bowel, wherein said GLP-1/GLP-2 dual agonist is administered to said patient within about 7 days of said surgical resection.

In one aspect the composition comprising the GLP-1/GLP-2 dual agonist is administered to said patient within about 6, 5, 4, 3, 2 or 1 day of said surgical resection.

In one aspect the composition comprising the GLP-1/GLP-2 dual agonist is administered to said patient within about 48 hours after surgery, about 44 hours after surgery, about 40 hours after surgery, about 36 hours after surgery, about 32 hours after surgery, about 32 hours after surgery, about 28 hours after surgery, about 24 hours after surgery, about 20 hours after surgery, about 16 hours after surgery, about 12 hours after surgery, about 8 hours after surgery, about 4 hours after surgery, about three hours after surgery, about two hours after surgery, or about one hour after surgery, preferably within 24 hours of said resection.

In one aspect the dual agonist may also be administered to said patient before surgery. For example, the dual agonist may be administered about 7, 6, 5, 4, 3, 2, or 1 day before surgery, for example 48 hours before surgery, about 44 hours before surgery, about 40 hours before surgery, about 36 hours before surgery, about 32 hours before surgery, about

32 hours before surgery, about 28 hours before surgery, about 24 hours before surgery, about 20 hours before surgery, about 16 hours before surgery, about 12 hours before surgery, about 8 hours before surgery, about 4 hours before surgery, about three hours before surgery, about two hours before surgery, or about one hour before surgery.

In a further aspect the patient may receive enteral nutrition after said surgical resection, for example within about 48 hours of said surgical resection.

After surgery, the dual agonist may be administered as a single dose administration. Alternatively, the dual agonist may be administered as a multi dose administration.

The inventors have found that the composition comprising a GLP-1/GLP-2 dual agonist is efficient for treatment of diarrhea, loose stools, urgency, incontinence, high fecal output and/or high stomal output over a long period of time, such as longer than 7 days, 10 days, 14 days, 1 month, 2 months, 3 months or more, such as treatment of a chronic condition or disorder. An advantage of the present invention is that efficiency of the treatment and/or prevention with said composition is constant, i.e. the efficiency of the treatment is persistent. Thus, in some embodiments the composition is administered to the patient for a period of at least 7 days, such as at least 10 days, at least 14 days, at least 1 month, at least 2 months or at least 3 months.

A therapeutic kit is disclosed comprising a dual agonist or composition comprising the dual agonist for use in the treatment of a patient who has undergone surgical resection of the bowel.

**GLP-1/GLP-2 dual agonist**

The GLP-1/GLP-2 dual agonist used in the present invention is a peptide.

Described herein is a GLP-1/GLP-2 dual agonist represented by the formula: $R^1$-X*-U-$R^2$, wherein:

$R^1$ is hydrogen (Hy), $C_{1-4}$ alkyl (e.g. methyl), acetyl, formyl, benzoyl or trifluoroacetyl;

$R^2$ is $NH_2$ or OH;

X* is a peptide of formula I:

H-X2-EG-X5-F-X7-X8-E-X10-X11-TIL-X15-X16-X17-A-X19-X20-X21-FI-X24-WL-X27-X28-X29-KIT-X33 (I) (SEQ ID NO 1)

wherein:

X2 is Aib or G; X5 is T or S; X7 is T or S; X8 is S, E or D; X10 is L, M, V or Ψ; X11 is A, N or S; X15 is D or E; X16 is G, E, A or Ψ; X17 is Q, E, K, L or Ψ; X19 is A, V or S; X20 is R, K or Ψ; X21 is D, L or E; X24 is A, N or S; X27 is I, Q, K, H or Y; X28 is Q, E, A, H, Y, L, K, R or S; X29 is H, Y, K or Q; X33 is D or E;

U is absent or a sequence of 1-15 residues each independently selected from K, k, E, A, T, I, Land Ψ;

the molecule contains one and only one Ψ, wherein Ψ is a residue of K, k, R, Orn, Dap or Dab in which the side chain is conjugated to a substituent having the formula $Z^1$- or $Z^1$-$Z^2$-, wherein

$Z^1$- is $CH_3-(CH_2)_{10-22}-(CO)-$ or $HOOC-(CH_2)_{10-22}-(CO)-$; and

-$Z^2$- is selected from -$Z^{S1}$-, -$Z^{S1}$-$Z^{S2}$-, -$Z^{S2}$-$Z^{S1}$, -$Z^{S2}$-, -$Z^{S3}$-, -$Z^{S1}Z^{S3}$-, -$Z^{S2}Z^{S3}$-, -$Z^{S3}Z^{S1}$-, - $Z^{S3}Z^{S2}$-, -$Z^{S1}Z^{S2}Z^{S3}$-, -$Z^{S1}Z^{S3}Z^{S2}$-, -$Z^{S2}Z^{S1}Z^{S3}$-, -$Z^{S2}Z^{S3}Z^{S1}$-, -$Z^{S3}Z^{S1}Z^{S2}$-, -$Z^{S3}Z^{S2}Z^{S1}$-, - $Z^{S2}Z^{S3}Z^{S2}$- wherein

$Z^{S1}$ is isoGlu, β-Ala, isoLys, or 4-aminobutanoyl;

$Z^{S2}$ is -$(Peg3)_m$- where m is 1, 2, or 3; and

-$Z^{S3}$- is a peptide sequence of 1-6 amino acid units independently selected from the group consisting of A, L, S, T, Y, Q, D, E, K, k, R, H, F and G;

and wherein at least one of X5 and X7 is T;

or a pharmaceutically acceptable salt or solvate thereof.

The various amino acid positions in peptide X* of the formulae provided here are numbered according to their linear position from N- to C-terminus in the amino acid chain.

In the present context, β-Ala and 3-Aminopropanoyl are used interchangeably.

Dual agonists having aspartic acid (Asp, D) at position 3 and glycine (Gly) in position 4 can be very potent agonists at the GLP-1 and GLP-2 receptors. However, this combination of substitutions results in compounds which are unstable and may not be suitable for long term storage in aqueous solution. Without wishing to be bound by theory, it is believed that the Asp at position 3 may isomerise to iso-Asp via a cyclic intermediate formed between the carboxylic acid functional group of its side chain and the backbone nitrogen atom of the residue at position 4.

It has now been found that molecules having glutamic acid (Glu, E) at position 3 instead of Asp are much less susceptible to such reactions and hence may be considerably more stable when stored in aqueous solution. However, replacement of Asp with Glu at position 3 in molecules having a lipophilic substituent in the middle portion of the peptide (e.g. at or near to positions 16 and 17) tends to reduce the potency at one or both of the GLP-2 receptor and the GLP-1 receptor, even though Glu is present at position 3 of the native GLP-1 molecule. Simultaneously incorporating a Thr residue at one or both of positions 5 and 7 appears to compensate for some or all of the lost potency. It is believed that further improvements in potency are also provided by incorporation of His (H), Tyr (Y), Lys (K) or Gln (Q) at position 29 instead of the Gly (G) and Thr (T) residues present in wild type human GLP-1 and 2 respectively.

In some instances of formula I:

X2 is Aib or G; X5 is T or S; X7 is T or S; X8 is S; X10 is L or Ψ; X11 is A or S; X15 is D or E; X16 is G, E, A or Ψ; X17 is Q, E, K, L or Ψ; X19 is A or S; X20 is R or Ψ; X21 is D, L or E; X24 is A; X27 is I, Q, K, or Y; X28 is Q, E, A, H, Y, L, K, R or S; X29 is H, Y or Q; and X33 is D or E.

Where Ψ is not at X16 or X17, it may be desirable that X16 is E and X17 is Q.

X11 may be A and X15 may be D. X11 may be S and X15 may be E. X11 may be A and X15may be E.

X27 may be I.

X29 may be H. X28 may be A and X29 may be H, or X28 may be E and X29may be H.

X29 may be Q and optionally X27 is Q.

The residues at X27-X29 may have a sequence selected from:

IQH; IEH IAH; IHH; IYH; ILH; IKH; IRH; ISH; QQH; YQH; KQH; IQQ; IQY; IQT; and IAY.

X* may be a peptide of formula II (SEQ ID NO 2):

H-X2-EG-X5-F-X7-SELATILD-X16-X17-AAR-X21-FIAWLI-X28-X29-KITD (II)

wherein: X2 is Aib or G; X5 is T or S; X7 is T or S; X16 is G or Ψ; X17 is Q, E, K, L or Ψ; X21 is D or L; X28 is Q, E, A, H, Y, L, K, R or S; X29 is H, Y or Q.

In some instances of Formula I or Formula II, X16 is Ψ and X17 is Q, E, K or L. For example, X17 may be Q, or X17 may be selected from E, K and L. X16 may be G and X17may be Ψ.

It may be desirable that X21 is D.

X28 may be selected from Q, E and A, e.g. it may be Q or E. In some residue combinations, Q may be preferred. In others, E may be preferred, including but not limited to when X16 is G and X17 is Ψ. Alternatively, X28 may be selected from A, H, Y, L, K, R and S.

X* may be a peptide of formula III (SEQ ID NO 3):

H[Aib]EG-X5-F-X7-SE-X10-ATILD-X16-X17-AA-X20-X21-FIAWLI-X28-X29-KITD (III)

wherein:

X5 is T or S; X7 is T or S; X10 is L or Ψ; X16 is G, E, A or Ψ; X17 is Q, E, K, L or Ψ; X20 is R or Ψ; X21 is D or L; X28 is E, A or Q; X29 is H, Y or Q; and at least one of X5 and X7 is T.

X* may be a peptide of formula IV (SEQ ID NO 4):

H[Aib]EG-X5-F-X7-SELATILD-X16-X17-AAR-X21-FIAWLI-X28-X29-KITD (IV)

wherein:

X5 is T or S; X7 is T or S; X16 is G or Ψ; X17 is E, K, L or Ψ; X21 is D or L; X28 is E or A; X29 is H, Y or Q; and at least one of X5 and X7 is T.

In some instances of any of formulae I to IV, X16 is Ψ and X17 is E, K or L.

In other instances of formula I to IV, X16 is G and X17 is Ψ.

In either case, the following combinations of residues may also be included:

X21 is D and X28 is E; X21 is D and X28 is A; X21 is L and X28 is E; or X21 is L and X28 is A.

X* may be a peptide of formula V (SEQ ID NO 5):

H[Aib]EG-X5-F-X7-SELATILD-Ψ-QAARDFIAWLI-X28-X29-KITD (V)

wherein

X5 is T or S; X7 is T or S; X28 is Q, E, A, H, Y, L, K, R or S, , e.g. Q, E, A, H, Y or L;
X29 is H, Y or Q; and at least one of X5 and X7 is T.

In some instances of formula III, X28 is Q or E. In some instances of formula III, X28 is Q. X28 may be A, H, Y, L, K, R or S, e.g. A, H, Y or L.
In any of the formulae described above, the dual agonist contains one of the following combinations of residues: X5 is S and X7 is T; X5 is T and X7 is S; or X5 is T and X7 is T.
It may be preferred that X5 is S and X7 is T, or X5 is T and X7 is T.
In any of the formulae described above, it may be desirable that X29 is H.
Ψ may be a Lys residue whose side chain is conjugated to the substituent $Z^1$- or $Z^1$-$Z^2$-.
$Z^1$-, alone or in combination with -$Z^2$-, may be dodecanoyl, tetradecanoyl, hexadecanoyl, octadecanoyl or eicosanoyl.
$Z^1$-, alone or in combination with -$Z^2$-, may be:
13-carboxytridecanoyl, i.e. HOOC-$(CH_2)_{12}$-(CO)-; 15-carboxypentadecanoyl, i.e. HOOC-$(CH_2)_{14}$-(CO)-; 17-carboxyheptadecanoyl, i.e. HOOC-$(CH_2)_{16}$-(CO)-; 19-carboxynonadecanoyl, i.e. HOOC-$(CH_2)_{18}$-(CO)-; or 21-carboxyheneicosanoyl, i.e. HOOC-$(CH_2)_{20}$-(CO)-.
$Z^2$ may be absent.
$Z^2$ may comprise $Z^{S1}$ alone or in combination with $Z^{S2}$ and/or $Z^{S3}$.
In such instances:
-$Z^{S1}$- is isoGlu, β-Ala, isoLys, or 4-aminobutanoyl; -$Z^{S2}$-, when present, is -$(Peg3)_m$- where m is 1, 2, or 3; and -$Z^{S3}$- is a peptide sequence of 1-6 amino acid units independently selected from the group consisting of A, L, S, T, Y, Q, D, E, K, k, R, H, F and G, such as the peptide sequence KEK.
$Z^2$ may have the formula -$Z^{S1}$-$Z^{S3}$-$Z^{S2}$-, where $Z^{S1}$ is bonded to $Z^1$ and $Z^{S2}$ is bonded to the side chain of the amino acid component of Ψ.
Thus, -$Z^2$- may be: isoGlu(Peg $3)_{0-3}$; β-Ala(Peg $3)_{0-3}$; isoLys$(Peg3)_{0-3}$; or 4-aminobutanoyl$(Peg3)_{0-3}$.
-$Z^2$- may be: isoGlu-KEK-$(Peg3)_{0-3}$.
Specific examples of the substituent $Z^1$-$Z^2$- are set out below. $Z^1$-$Z^2$- may be [17-carboxy-heptadecanoyl]-isoGlu. For example, Ψ may be K([17-carboxy-heptadecanoyl]-isoGlu). $Z^1$-$Z^2$ - may be:

[17-Carboxy-heptadecanoyl]-isoGlu-KEK-Peg3-; [17-carboxy-heptadecanoyl]-isoGlu-Peg3-; [19-Carboxy-nonadecanoyl]-isoGlu-; [19-Carboxy-nonadecanoyl]-isoGlu-KEK-;

[19-Carboxy-nonadecanoyl]-isoGlu-KEK-Peg3-; [19-carboxy-nonadecanoyl]-isoGlu-KEK-Peg3-Peg3-; [19-carboxy-nonadecanoyl]-isoGlu-Peg3-Peg3-; [19-carboxy-nonadecanoyl]-isoLys-Peg3-Peg3-Peg3-; [Hexadecanoyl]-βAla-; [Hexadecanoyl]-isoGlu-; or Octadecanoyl-.

For example, Ψ may be:
K([17-Carboxy-heptadecanoyl]-isoGlu-KEK-Peg3); K([17-carboxy-heptadecanoyl]-isoGlu-Peg3); K([19-Carboxy-nonadecanoyl]-isoGlu); K([19-Carboxy-nonadecanoyl]-isoGlu-KEK); K([19-Carboxy-nonadecanoyl]-isoGlu-KEK-Peg3); K([19-carboxy-nonadecanoyl]-isoGlu-KEK-Peg3-Peg3); K([19-carboxy-nonadecanoyl]-isoGlu-Peg3-Peg3); K([19-carboxynonadecanoyl]-isoLys-Peg3-Peg3-Peg3); K([Hexadecanoyl]- βAla-; K([Hexadecanoyl]-isoGlu); or K(Octadecanoyl).
When present, U represents a peptide sequence of 1-15 residues each independently selected from K (i.e. L-lysine), k (i.e. D-lysine) E (Glu), A (Ala), T (Thr), I (Ile), L (Leu) and Ψ. For example, U may be 1-10 amino acids in length, 1-7 amino acids in length, 3-7 amino acids in length, 1-6 amino acids in length, or 3-6 amino acids in length.
Typically U includes at least one charged amino acid (K, k or E) and preferably two or more charged amino acids. It may include at least 2 positively charged amino acids (K or k), or at least 1 positively charged amino acid (K or k) and at least one negatively charged amino acid (E). All amino acid residues of U (except for Ψ, if present) may be charged. For example, U may be a chain of alternately positively and negatively charged amino acids.
U may comprise residues selected only from K, k, E and Ψ.
U may comprise residues selected only from K, k, and Ψ.
When U comprises only lysine residues (whether K or k), all residues may have an L-configuration or all may have a D-configuration. Examples include $K_{1-15}$, $K_{1-10}$ and $K_{1-7}$, e.g., $K_3$, $K_4$, $K_5$, $K_6$ and $K_7$, especially $K_5$ and $K_6$. Further examples include $k_{1-15}$, $k_{1-10}$ and $k_{1-7}$, e.g. $k_3$, $k_4$, $k_5$, $k_6$ and $k_7$, especially $k_5$ and $k_6$.
Further examples of peptide sequences U include KEK, EKEKEK (SEQ ID NO 7), EkEkEk (SEQ ID NO 8), AKAAEK (SEQ ID NO 9), AKEKEK (SEQ ID NO 10) and ATILEK (SEQ ID NO 11).
In any case, one of those residues may be exchanged for Ψ. Where the sequence U contains a residue Ψ, it may be desirable that the C-terminal residue of U is Ψ. Thus, further examples of sequences U include $K_{1-14}$-Ψ, $K_{1-9}$-Ψ and $K_{1-6}$-

$\Psi$, e.g., $K_2$-$\Psi$, $K_3$-$\Psi$, $K_4$-$\Psi$, $K_5$-$\Psi$ and $K_6$-$\Psi$, especially $K_4$-$\Psi$ and $K_5$.-$\Psi$. Yet further examples include $k_{1-14}$-$\Psi$, $k_{1-9}$-$\Psi$, and $k_{1-6}$-$\Psi$, e.g. $k_2$-$\Psi$, $k_3$-$\Psi$, $k_4$-$\Psi$, $k_5$-$\Psi$ and $k_6$-$\Psi$ especially $k_4$-$\Psi$ and $k_5$-$\Psi$. Yet further examples include KE$\Psi$, EKEKE$\Psi$ (SEQ ID NO 12), EkEkE$\Psi$ (SEQ ID NO 13), AKAAE$\Psi$ (SEQ ID NO 14), AKEKE$\Psi$ (SEQ ID NO 15) and ATILE$\Psi$ (SEQ ID NO 16).

U may be absent.

$R^1$ may be Hy and/or $R^2$ may be OH.

Substituents

The dual agonist may contain a residue $\Psi$ which comprises a residue of Lys, Arg, Orn, Dap or Dab in which the side chain is conjugated to a substituent $Z^1$- or $Z^1$-$Z^2$- wherein $Z^1$ represents a moiety $CH_3$-$(CH_2)_{10-22}$-(CO)- or HOOC-$(CH_2)_{10-22}$-(CO)- and $Z^2$ when present represents a spacer.

The spacer $Z^2$ is selected from -$Z^{S1}$-, -$Z^{S1}$-$Z^{S2}$-, -$Z^{S2}$-$Z^{S1}$, -$Z^{S2}$-, -$Z^{S3}$-, -$Z^{S1}Z^{S3}$-, -$Z^{S2}Z^{S3}$-, - $Z^{S3}Z^{S1}$-, -$Z^{S3}Z^{S2}$-, -$Z^{S1}Z^{S2}Z^{S3}$-, -$Z^{S1}Z^{S3}Z^{S2}$-, -$Z^{S2}Z^{S1}Z^{S3}$-, -$Z^{S2}Z^{S3}Z^{S1}$-, -$Z^{S3}Z^{S1}Z^{S2}$-, -$Z^{S3}Z^{S2}Z^{S1}$-, $Z^{S2}Z^{S3}Z^{S2}$- wherein

$Z^{S1}$ is isoGlu, $\beta$-Ala, isoLys, or 4-aminobutanoyl;

$Z^{S2}$ is -$(Peg3)_m$- where m is 1, 2, or 3; and

$Z^{S3}$- is a peptide sequence of 1-6 amino acid units selected from the group consisting of A, L, S, T, Y, Q, D, E, K, k, R, H, F and G.

In some embodiments, $Z^2$ is a spacer of the formula -$Z^{S1}$-, -$Z^{S1}$-$Z^{S2}$-, -$Z^{S2}$-$Z^{S1}$, or $Z^{S2}$, where - $Z^{S1}$- is isoGlu, $\beta$-Ala, isoLys, or 4-aminobutanoyl; and -$Z^{S2}$- is -$(Peg3)_m$- where m is 1, 2, or 3.

Without wishing to be bound by theory, it is believed that the hydrocarbon chain of $Z^1$ binds albumin in the blood stream, thus shielding the dual agonists of the present invention from enzymatic degradation, which can enhance the half-life of the dual agonists.

The substituent may also modulate the potency of the dual agonists, with respect to the GLP-2 receptor and/or the GLP-1 receptor.

The substituent $Z^1$- or $Z^1$-$Z^2$- is conjugated to the functional group at the distal end of the side-chain from the alpha-carbon of the relevant amino acid residue. The normal ability of the amino acid (Lys, Arg, Orn, Dab, Dap) side-chain in question to participate in interactions mediated by that functional group (e.g. intra- and inter-molecular interactions) may therefore be reduced or completely eliminated by the presence of the substituent. Thus, the overall properties of the dual agonist may be relatively insensitive to changes in the actual amino acid conjugated to the substituent. Consequently, it is believed that any of the residues Lys, Arg, Orn, Dab, or Dap may be present at any position where $\Psi$ is permitted. However, in certain embodiments, it may be advantageous that the amino acid to which the substituent is conjugated is Lys or Orn.

The moiety $Z^1$ may be covalently bonded to the functional group in the amino acid side-chain, or alternatively may be conjugated to the amino acid side-chain functional group via a spacer $Z^2$.

The term "conjugated" is used here to describe the covalent attachment of one identifiable chemical moiety to another, and the structural relationship between such moieties. It should not be taken to imply any particular method of synthesis.

The bonds between $Z^1$, $Z^{S1}$, $Z^{S2}$, $Z^{S3}$ and the amino acid side chain to which the substituent is bound (collectively referred to herein as $\Psi$) are peptidic. In other words, the units may be joined by amide condensation reactions.

$Z^1$ comprises a hydrocarbon chain having from 10 to 24 carbon (C) atoms, such as from 10 to 22 C atoms, e.g. from 10 to 20 C atoms. Preferably, it has at least 10 or at least 11 C atoms, and preferably it has 20 C atoms or fewer, e.g. 18 C atoms or fewer. For example, the hydrocarbon chain may contain 12, 13, 14, 15, 16, 17, 18, 19 or 20 carbon atoms. For example, it may contain 18 or 20 carbon atoms.

$Z^1$ may be a group selected from dodecanoyl, tetradecanoyl, hexadecanoyl, octadecanoyl and eicosanoyl, preferably hexadecanoyl, octadecanoyl or eicosanoyl, more preferably octadecanoyl or eicosanoyl.

Alternative $Z^1$ groups are derived from long-chain saturated $\alpha,\omega$-dicarboxylic acids of formula HOOC-$(CH_2)_{12-22}$-COOH, preferably from long-chain saturated $\alpha,\omega$-dicarboxylic acids having an even number of carbon atoms in the aliphatic chain. For example, $Z^1$ may be:

13-carboxytridecanoyl, i.e. HOOC-$(CH_2)_{12}$-(CO)-;

15-carboxypentadecanoyl, i.e. HOOC-$(CH_2)_{14}$-(CO)-;

17-carboxyheptadecanoyl, i.e. HOOC-$(CH_2)_{16}$-(CO)-;

19-carboxynonadecanoyl, i.e. HOOC-$(CH_2)_{18}$-(CO)-; or

21-carboxyheneicosanoyl, i.e. HOOC-$(CH_2)_{20}$-(CO)-.

As mentioned above, $Z^1$ may be conjugated to the amino acid side-chain by a spacer $Z^2$. When present, the spacer is attached to $Z^1$ and to the amino acid side-chain.

The spacer $Z^2$ **has the** $-Z^{S1}-$, $-Z^{S1}-Z^{S2}-$, $-Z^{S2}-Z^{S1}$, $-Z^{S2}-$, $-Z^{S3}-$, $-Z^{S1}Z^{S3}-$, $-Z^{S2}Z^{S3}-$, $-Z^{S3}Z^{S1}-$, - $Z^{S3}Z^{S2}-$, $-Z^{S1}Z^{S2}Z^{S3}-$, $-Z^{S1}Z^{S3}Z^{S2}-$, $-Z^{S2}Z^{S1}Z^{S3}-$, $-Z^{S2}Z^{S3}Z^{S1}-$, $-Z^{S3}Z^{S1}Z^{S2}-$, $-Z^{S3}Z^{S2}Z^{S1}-$, $Z^{S2}Z^{S3}Z^{S2}-$; where

- $Z^{S1}$- is isoGlu, β-Ala, isoLys, or 4-aminobutanoyl;
- $Z^{S2}$- is -$(Peg3)_m$- where m is 1, 2, or 3; and
- $Z^{S3}$- is a peptide sequence of 1-6 amino acid units independently selected from the group consisting of A (Ala), L (Leu), S (Ser), T (Thr), Y (Tyr), Q (Gln), D (Asp), E (Glu), K (L-Lys), k (D-Lys), R (Arg), H (His), F (Phe) and G (Gly).

The terms "isoGlu" and "isoLys" indicate residues of amino acids which participate in bonds via their side chain carboxyl or amine functional groups. Thus isoGlu participates in bonds via its alpha amino and side chain carboxyl group, while isoLys participates via its carboxyl and side chain amino groups. In the context of the present specification, the terms "γ-Glu" and "isoGlu" are used interchangeably.

The term Peg3 is used to refer to an 8-amino-3,6-dioxaoctanoyl group.

$Z^{S3}$ may, for example, be 3 to 6 amino acids in length, i.e. 3, 4, 5 or 6 amino acids in length.

The amino acids of $Z^{S3}$ may be independently selected from K, k, E, A, T, I and L, e.g. from K, k, E and A, e.g. from K, k and E.

Typically $Z^{S3}$ includes at least one charged amino acid (K, k, R or E, e.g. K, k or E) and preferably two or more charged amino acids. It may include at least 2 positively charged amino acids (K, k or R, especially K or k), or at least 1 positively charged amino acid (K, k or R, especially K or k) and at least one negatively charged amino acid (E). All amino acid residues of $Z^{S3}$ may be charged. For example, $Z^{S3}$ may be a chain of alternately positively and negatively charged amino acids.

Examples of $Z^{S3}$ moieties include KEK, EKEKEK (SEQ ID NO 7), kkkkkk (SEQ ID NO 6), EkEkEk (SEQ ID NO 8), AKAAEK (SEQ ID NO 9), AKEKEK (SEQ ID NO 10) and ATILEK (SEQ ID NO 11).

Without being bound by theory, it is believed that the incorporation of $Z^{S3}$ into the linker between the fatty acid chain and the peptide backbone may increase the half-life of the dual agonist by enhancing its affinity for serum albumin.

Certain preferred substituents $Z^1$- and $Z^1$-$Z^2$- include:
[Hexadecanoyl], [Octadecanoyl], [17-Carboxy-heptadecanoyl], [19-Carboxynonadecanoyl], [Hexadecanoyl]-isoGlu, [Octadecanoyl]-isoGlu, [Hexadecanoyl]-βAla, [Octadecanoyl]-βAla, [Hexadecanoyl]-isoGlu-Peg3, [Hexadecanoyl]-βAla-Peg3, [Hexadecanoyl]-isoGlu-Peg3-Peg3, [Hexadecanoyl]-βAla-Peg3-Peg3, [Hexadecanoyl]-βAla-Peg3-Peg3-Peg3, [Hexadecanoyl]-isoLys, [Hexadecanoyl]-[4-aminobutanoyl], [Hexadecanoyl]-isoLys-Peg3, [Hexadecanoyl]-[4-aminobutanoyl]-Peg3, [Hexadecanoyl]-isoLys-Peg3-Peg3, [Hexadecanoyl]-[4-aminobutanoyl]-Peg3-Peg3, [Hexadecanoyl]-isoLys-Peg3-Peg3-Peg3, [Hexadecanoyl]-[4-aminobutanoyl]-Peg3-Peg3-Peg3, [17-carboxy-Heptadecanoyl]-isoGlu, [19-carboxy-Nonadecanoyl]-isoGlu, [17-carboxy-Heptadecanoyl]-βAla, [19-carboxy-Nonadecanoyl]-βAla, [17-carboxy-Heptadecanoyl]-isoGlu-Peg3, [19-carboxy-Nonadecanoyl]-isoGlu-Peg3, [17-carboxy-Heptadecanoyl]-βAla-Peg3, [19-carboxy-Nonadecanoyl]-βAla-Peg3, [17-carboxy-Heptadecanoyl]-isoGlu-Peg3-Peg3, [19-carboxy-Nonadecanoyl]-isoGlu-Peg3-Peg3, [17-carboxy-Heptadecanoyl]-βAla-Peg3-Peg3, [19-carboxyNonadecanoyl]-βAla-Peg3-Peg3, [17-carboxy-Heptadecanoyl]-isoGlu-Peg3-Peg3-Peg3, [19-carboxy-Nonadecanoyl]-isoGlu-Peg3-Peg3-Peg3, [17-carboxy-Heptadecanoyl]-βAla-Peg3-Peg3-Peg3, [19-carboxy-Nonadecanoyl]-βAla-Peg3-Peg3-Peg3, [17-carboxy-Heptadecanoyl]-isoLys, [19-carboxy-Nonadecanoyl]-isoLys, [17-carboxy-Heptadecanoyl]-[4-aminobutanoyl], [19-carboxy-Nonadecanoyl]-[4-aminobutanoyl], [17-carboxy-Heptadecanoyl]-isoLys-Peg3, [19-carboxyNonadecanoyl]-isoLys-Peg3, [17-carboxy-Heptadecanoyl]-[4-aminobutanoyl]-Peg3, [19-carboxy-Nonadecanoyl]- [4-aminobutanoyl]-Peg3, [17-carboxy-Heptadecanoyl]-isoLys-Peg3-Peg3, [19-carboxy-Nonadecanoyl]-isoLys-Peg3-Peg3, [17-carboxy-Heptadecanoyl]-[4-aminobutanoyl]-Peg3-Peg3, [19-carboxy-Nonadecanoyl]-[4-aminobutanoyl]-Peg3-Peg3, [17-carboxy-Heptadecanoyl]-isoLys-Peg3-Peg3-Peg3, [19-carboxy-Nonadecanoyl]-isoLys-Peg3-Peg3-Peg3, [17-carboxy-Heptadecanoyl]-[4-aminobutanoyl]-Peg3-Peg3-Peg3, [19-carboxy-Nonadecanoyl]-[4-aminobutanoyl]-Peg3-Peg3-Peg3.

The substituent in the GLP-1/GLP-2 dual agonist of the invention is [17-Carboxy-heptadecanoyl]-isoGlu

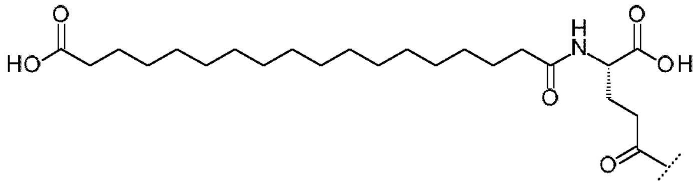

The dual agonist described herein may be any of the agonists as described in WO2018/104560.

For example, the GLP-1/GLP-2 dual agonist may be a compound represented by the formula: $R^1$-X*-U-$R^2$, wherein:

$R^1$ is hydrogen (Hy), $C_{1-4}$ alkyl (e.g. methyl), acetyl, formyl, benzoyl or trifluoroacetyl;

$R^2$ is $NH_2$ or OH;

X* is a peptide of formula I (SEQ ID NO 1):
H-X2-EG-X5-F-X7-X8-E-X10-X11-TI L-X15-X16-X17-A-X19-X20-X21-FI-X24-WL-X27-X28-X29-KIT-X33 (I), wherein

X2 is Aib or G; X5 is S or T; X7 is S or T; X8 is S, E or D; X10 is L, M or V; X11 is A, N or S; X15 is 0 or E; X16 is E, A or G; X17 is Q, E, L or K; X19 is A, V or S; X20 is R or K; X21 is D, L or E; X24 is A, N or S; X27 is I, Y, Q, H or K; X28 is A, E, H, Y, L, K, Q, R or S; X29 is H, Y, K or Q; X33 is D or E;

U is absent or a sequence of 1-15 residues, each independently selected from K and k;

and wherein at least one of X5 and X7 is T;

or a pharmaceutically acceptable salt or solvate thereof.

<u>Peptide sequences</u>

The peptide X* or the peptide X*-U may have the sequence:

H[Aib]EGTFSSELATILDΨEAARDFIAWLIEHKITD (SEQ ID NO 17);
H[Aib]EGSFTSELATILDΨEAARDFIAWLIEHKITD (SEQ ID NO 18);
H[Aib]EGTFTSELATILDΨEAARDFIAWLIEHKITD (SEQ ID NO 19);
H[Aib]EGTFSSELATILDΨKAARDFIAWLIEHKITD (SEQ ID NO 20);
H[Aib]EGSFTSELATILDΨKAARDFIAWLIEHKITD (SEQ ID NO 21);
H[Aib]EGTFTSELATILDΨKAARDFIAWLIEHKITD (SEQ ID NO 22);
H[Aib]EGTFSSELATILDGΨAARDFIAWLIEHKITD (SEQ ID NO 23);
H[Aib]EGSFTSELATILDGΨAARDFIAWLIEHKITD (SEQ ID NO 24);
H[Aib]EGTFTSELATILDGΨAARDFIAWLIEHKITD (SEQ ID NO 25);
H[Aib]EGTFSSELATILDΨLAARDFIAWLIEHKITD (SEQ ID NO 26);
H[Aib]EGSFTSELATILDΨLAARDFIAWLIEHKITD (SEQ ID NO 27);
H[Aib]EGTFTSELATILDΨLAARDFIAWLIEHKITD (SEQ ID NO 28);
H[Aib]EGTFSSELATILDΨLAARDFIAWLIAHKITD (SEQ ID NO 29);
H[Aib]EGSFTSELATILDΨLAARDFIAWLIAHKITD (SEQ ID NO 30);
H[Aib]EGTFTSELATILDΨLAARDFIAWLIAHKITD (SEQ ID NO 31);
H[Aib]EGTFTSELATILDΨEAARLFIAWLIEHKITD (SEQ ID NO 32);
H[Aib]EGTFSSELATILDΨQAARDFIAWLIQHKITD (SEQ ID NO 33);
H[Aib]EGSFTSELATILDΨQAARDFIAWLIQHKITD (SEQ ID NO 34);
H[Aib]EGTFTSELATILDΨQAARDFIAWLIQHKITD (SEQ ID NO 35);
H[Aib]EGTFSSELATILDΨQAARDFIAWLIEHKITD (SEQ ID NO 36);
H[Aib]EGTFSSELATILDΨQAARDFIAWLIAHKITD (SEQ ID NO 37);
H[Aib]EGSFTSELATILDΨQAARDFIAWLIAHKITD (SEQ ID NO 38);
H[Aib]EGTFTSELATILDΨQAARDFIAWLIAHKITD (SEQ ID NO 39);
H[Aib]EGSFTSELATILDΨQAARDFIAWLIEHKITD (SEQ ID NO 40);
H[Aib]EGTFTSELATILDΨQAARDFIAWLIEHKITD (SEQ ID NO 41);

H[Aib]EGSFTSELATILDΨQAARDFIAWLIHHKITD (SEQ ID NO 42);
H[Aib]EGSFTSELATILDΨQAARDFIAWLIYHKITD (SEQ ID NO 43);
H[Aib]EGSFTSELATILDΨQAARDFIAWLILHKITD (SEQ ID NO 44);
H[Aib]EGSFTSELATILDΨQAARDFIAWLIKHKITD (SEQ ID NO 45);
H[Aib]EGSFTSELATILDΨQAARDFIAWLIRHKITD (SEQ ID NO 46);
H[Aib]EGSFTSELATILDΨQAARDFIAWLISHKITD (SEQ ID NO 47);
H[Aib]EGSFTSELATILDΨQAARDFIAWLQQHKITD (SEQ ID NO 48);
H[Aib]EGSFTSELATILDΨQAARDFIAWLYQHKITD (SEQ ID NO 49);
H[Aib]EGSFTSELATILDΨQAARDFIAWLKQHKITD (SEQ ID NO 50);
H[Aib]EGSFTSELATILDΨQAARDFIAWLIQQKITD (SEQ ID NO 51);
H[Aib]EGSFTSELATILDΨQAARDFIAWLIQYKITD (SEQ ID NO 52);
H[Aib]EGTFSSELSTILEΨQASREFIAWLIAYKITE (SEQ ID NO 53);
H[Aib]EGTFSSELATILDEQAARDFIAWLIAHKITDkkkkkΨ (SEQ ID NO 54);
H[Aib]EGTFTSELATILDEQAARDFIAWLIAHKITDkkkkkΨ (SEQ ID NO 55);
H[Aib]EGSFTSELATILDEQAARDFIAWLIEHKITDkkkkkΨ (SEQ ID NO 56);
H[Aib]EGSFTSEΨATILDEQAARDFIAWLIEHKITD (SEQ ID NO 57);
H[Aib]EGSFTSELATILEGΨAARDFIAWLIEHKITD (SEQ ID NO 58);
H[Aib]EGSFTSELATILDEQAAΨDFIAWLIEHKITD (SEQ ID NO 59);
H[Aib]EGTFTSELATILDEQAAΨDFIAWLIEHKITD (SEQ ID NO 60);
H[Aib]EGTFTSEψATILDEQAARDFIAWLIEHKITD (SEQ ID NO 61);
H[Aib]EGSFTSELATILDAψAARDFIAWLIEHKITD (SEQ ID NO 62); or
H[Aib]EGSFTSELATILDAKAAψDFIAWLIEHKITD (SEQ ID NO 63).

The peptide X* or the peptide X*-U may have the sequence H[Aib]EGSFTSELATILDΨQAARDFIAWLIQHKITD (SEQ ID NO 34).

The peptide X* or the peptide X*-U may have the sequence:

H[Aib]EGTFSSELATILD[K*]EAARDFIAWLIEHKITD (SEQ ID NO 64);
H[Aib]EGSFTSELATILD[K*]EAARDFIAWLIEHKITD (SEQ ID NO 65);
H[Aib]EGTFTSELATILD[K*]EAARDFIAWLIEHKITD (SEQ ID NO 66);
H[Aib]EGTFSSELATILD[K*]KAARDFIAWLIEHKITD (SEQ ID NO 67);
H[Aib]EGSFTSELATILD[K*]KAARDFIAWLIEHKITD (SEQ ID NO 68);
H[Aib]EGTFTSELATILD[K*]KAARDFIAWLIEHKITD (SEQ ID NO 69);
H[Aib]EGTFSSELATILDG[K*]AARDFIAWLIEHKITD (SEQ ID NO 70);
H[Aib]EGSFTSELATILDG[K*]AARDFIAWLIEHKITD (SEQ ID NO 71);
H[Aib]EGTFTSELATILDG[K*]AARDFIAWLIEHKITD (SEQ ID NO 72);
H[Aib]EGTFSSELATILD[K*]LAARDFIAWLIEHKITD (SEQ ID NO 73);
H[Aib]EGSFTSELATILD[K*]LAARDFIAWLIEHKITD (SEQ ID NO 74);
H[Aib]EGTFTSELATILD[K*]LAARDFIAWLIEHKITD (SEQ ID NO 75);
H[Aib]EGTFSSELATILD[K*]LAARDFIAWLIAHKITD (SEQ ID NO 76);
H[Aib]EGSFTSELATILD[K*]LAARDFIAWLIAHKITD (SEQ ID NO 77);
H[Aib]EGTFTSELATILD[K*]LAARDFIAWLIAHKITD (SEQ ID NO 78);
H[Aib]EGTFTSELATILD[K*]EAARLFIAWLIEHKITD (SEQ ID NO 79);
H[Aib]EGTFSSELATILD[K*]QAARDFIAWLIQHKITD (SEQ ID NO 80);
H[Aib]EGSFTSELATILD[K*]QAARDFIAWLIQHKITD (SEQ ID NO 81);
H[Aib]EGTFTSELATILD[K*]QAARDFIAWLIQHKITD (SEQ ID NO 82);
H[Aib]EGTFSSELATILD[K*]QAARDFIAWLIEHKITD (SEQ ID NO 83);
H[Aib]EGTFSSELATILD[K*]QAARDFIAWLIAHKITD (SEQ ID NO 84);
H[Aib]EGSFTSELATILD[K*]QAARDFIAWLIAHKITD (SEQ ID NO 85);
H[Aib]EGTFTSELATILD[K*]QAARDFIAWLIAHKITD (SEQ ID NO 86);
H[Aib]EGSFTSELATILD[K*]QAARDFIAWLIEHKITD (SEQ ID NO 87);
H[Aib]EGTFTSELATILD[K*]QAARDFIAWLIEHKITD (SEQ ID NO 88);
H[Aib]EGSFTSELATILD[K*]QAARDFIAWLIHHKITD (SEQ ID NO 89);
H[Aib]EGSFTSELATILD[K*]QAARDFIAWLIYHKITD (SEQ ID NO 90);
H[Aib]EGSFTSELATILD[K*]QAARDFIAWLILHKITD (SEQ ID NO 91);
H[Aib]EGSFTSELATILD[K*]QAARDFIAWLIKHKITD (SEQ ID NO 92);
H[Aib]EGSFTSELATILD[K*]QAARDFIAWLIRHKITD (SEQ ID NO 93);
H[Aib]EGSFTSELATILD[K*]QAARDFIAWLISHKITD (SEQ ID NO 94);

H[Aib]EGSFTSELATILD[K*]QAARDFIAWLQQHKITD (SEQ ID NO 95);
H[Aib]EGSFTSELATILD[K*]QAARDFIAWLYQHKITD (SEQ ID NO 96);
H[Aib]EGSFTSELATILD[K*]QAARDFIAWLKQHKITD (SEQ ID NO 97);
H[Aib]EGSFTSELATILD[K*]QAARDFIAWLIQQKITD (SEQ ID NO 98);
H[Aib]EGSFTSELATILD[K*]QAARDFIAWLIQYKITD (SEQ ID NO 99);
H[Aib]EGTFSSELSTILE[K*]QASREFIAWLIAYKITE (SEQ ID NO 100);
H[Aib]EGTFSSELATILDEQAARDFIAWLIAHKITDkkkkk[k*] (SEQ ID NO 101);
H[Aib]EGTFTSELATILDEQAARDFIAWLIAHKITDkkkkk[k*] (SEQ ID NO 102);
H[Aib]EGSFTSELATILDEQAARDFIAWLIEHKITDkkkkk[k*] (SEQ ID NO 103);
H[Aib]EGSFTSE[K*]ATILDEQAARDFIAWLIEHKITD (SEQ ID NO 104);
H[Aib]EGSFTSELATILEG[K*]AARDFIAWLIEHKITD (SEQ ID NO 105);
H[Aib]EGSFTSELATILDEQAA[K*]DFIAWLIEHKITD (SEQ ID NO 106);
H[Aib]EGTFTSELATILDEQAA[K*]DFIAWLIEHKITD (SEQ ID NO 107);
H[Aib]EGTFTSE[K*]ATILDEQAARDFIAWLIEHKITD (SEQ ID NO 108);
H[Aib]EGSFTSELATILDA[K*]AARDFIAWLIEHKITD (SEQ ID NO 109); or
H[Aib]EGSFTSELATILDAKAA[K*]DFIAWLIEHKITD (SEQ ID NO 110);

wherein K* or k* indicates an L or D lysine residue respectively in which the side chain is conjugated to the substituent $Z^1$- or $Z^1Z^2$-.

The peptide X* or the peptide X*-U may have the sequence H[Aib]EGSFTSELATILD[K*]QAARDFIAWLIQHKITD (SEQ ID NO 81).

For example, the peptide X* or the peptide X*-U may have the sequence:

H[Aib]EGTFSSELATILD[K([17-carboxy-heptadecanoyl]-isoGlu)]EAARDFIAWLIEHKITD (SEQ ID NO 111);

H[Aib]EGSFTSELATILD[K([17-carboxy-heptadecanoyl]-isoGlu)]EAARDFIAWLIEHKITD (SEQ ID NO 112);

H[Aib]EGTFTSELATILD[K([17-carboxy-heptadecanoyl]-isoGlu)]EAARDFIAWLIEHKITD (SEQ ID NO 113);

H[Aib]EGTFSSELATILD[K([17-carboxy-heptadecanoyl]-isoGlu)]KAARDFIAWLIEHKITD (SEQ ID NO 114);

H[Aib]EGSFTSELATILD[K([17-carboxy-heptadecanoyl]-isoGlu)]KAARDFIAWLIEHKITD (SEQ ID NO 115);

H[Aib]EGTFTSELATILD[K([17-carboxy-heptadecanoyl]-isoGlu)]KAARDFIAWLIEHKITD (SEQ ID NO 116);

H[Aib]EGTFSSELATILDG[K([17-carboxy-heptadecanoyl]-isoGlu)]AARDFIAWLIEHKITD (SEQ ID NO 117);

H[Aib]EGSFTSELATILDG[K([17-carboxy-heptadecanoyl]-isoGiu)]AARDFIAWLIEHKITD (SEQ ID NO 118);

H[Aib]EGTFTSELATILDG[K([17-carboxy-heptadecanoyl]-isoGlu)]AARDFIAWLIEHKITD (SEQ ID NO 119);

H[Aib]EGTFSSELATILD[K([17-carboxy-heptadecanoyl]-isoGlu)]LAARDFIAWLIEHKITD (SEQ ID NO 120);

H[Aib]EGSFTSELATILD[K([17-carboxy-heptadecanoyl]-isoGlu)]LAARDFIAWLIEHKITD (SEQ ID NO 121);

H[Aib]EGTFTSELATILD[K([17-carboxy-heptadecanoyl]-isoGlu)]LAARDFIAWLIEHKITD (SEQ ID NO 122);

H[Aib]EGTFSSELATILD[K([17-carboxy-heptadecanoyl]-isoGlu)]LAARDFIAWLIAHKITD (SEQ ID NO 123);

H[Aib]EGSFTSELATILD[K([17-carboxy-heptadecanoyl]-isoGlu)]LAARDFIAWLIAHKITD (SEQ ID NO 124);

H[Aib]EGTFTSELATILD[K([17-carboxy-heptadecanoyl]-isoGlu)]LAARDFIAWLIAHKITD (SEQ ID NO 125);

H[Aib]EGTFTSELATILD[K([17-carboxy-heptadecanoyl]-isoGlu)]EAARLFIAWLIEHKITD (SEQ ID NO 126);

H[Aib]EGTFSSELATILD[K([17-carboxy-heptadecanoyl]-isoGlu)]QAARDFIAWLIQHKITD (SEQ ID NO 127);

H[Aib]EGSFTSELATILD[K([17-carboxy-heptadecanoyl]-isoGlu)]QAARDFIAWLIQHKITD (SEQ ID NO 128);

H[Aib]EGTFTSELATILD[K([17-carboxy-heptadecanoyl]-isoGlu)]QAARDFIAWLIQHKITD (SEQ ID NO 129);

H[Aib]EGTFSSELATILD[K([17-carboxy-heptadecanoyl]-isoGlu)]QAARDFIAWLIEHKITD (SEQ ID NO 130);

H[Aib]EGTFSSELATILD[K([17-carboxy-heptadecanoyl]-isoGlu)]QAARDFIAWLIAHKITD (SEQ ID NO 131);

H[Aib]EGSFTSELATILD[K([17-carboxy-heptadecanoyl]-isoGlu)]QAARDFIAWLIAHKITD (SEQ ID NO 132);

H[Aib]EGTFTSELATILD[K([17-carboxy-heptadecanoyl]-isoGlu)]QAARDFIAWLIAHKITD (SEQ ID NO 133);

H[Aib]EGSFTSELATILD[K([17-carboxy-heptadecanoyl]-isoGlu)]QAARDFIAWLIEHKITD (SEQ ID NO 134);

H[Aib]EGTFTSELATILD[K([17-carboxy-heptadecanoyl]-isoGlu)]QAARDFIAWLIEHKITD (SEQ ID NO 135);

H[Aib]EGSFTSELATILD[K([17-carboxy-heptadecanoyl]-isoGlu)]QAARDFIAWLIHHKITD (SEQ ID NO 136);

H[Aib]EGSFTSELATILD[K([17-carboxy-heptadecanoyl]-isoGlu)]QAARDFIAWLIYHKITD (SEQ ID NO 137);

H[Aib]EGSFTSELATILD[K([17-carboxy-heptadecanoyl]-isoGlu)]QAARDFIAWLILHKITD (SEQ ID NO 138);

H[Aib]EGSFTSELATILD[K([17-carboxy-heptadecanoyl]-isoGlu)]QAARDFIAWLIKHKITD (SEQ ID NO 139);

H[Aib]EGSFTSELATILD[K([17-carboxy-heptadecanoyl]-isoGlu)]QAARDFIAWLIRHKITD (SEQ ID NO 140);

H[Aib]EGSFTSELATILD[K([17-carboxy-heptadecanoyl]-isoGlu)]QAARDFIAWLISHKITD (SEQ ID NO 141);

H[Aib]EGSFTSELATILD[K([Hexadecanoyl]-βAla)]QAARDFIAWLQQHKITD (SEQ ID NO 142);

H[Aib]EGSFTSELATILD[K([17-carboxy-heptadecanoyl]iso-Glu-Peg3)]QAARDFIAWLYQHKITD (SEQ ID NO 143);

H[Aib]EGSFTSELATILD[K([19-carboxy-nonadecanoyl]iso-Glu-Peg3-Peg3)]QAARDFIAWLKQHKITD (SEQ ID NO 144);

H[Aib]EGSFTSELATILD[K([19-carboxy-nonadecanoyl]iso-Lys-Peg3-Peg3-Peg3)]QAARDFIAWLIQQKITD (SEQ ID NO 145);

H[Aib]EGSFTSELATILD[K(Octadecanoyl)]QAARDFIAWLIQYKITD (SEQ ID NO 146);

H[Aib]EGTFSSELSTILE[K(Hexadecanoyl-isoGlu)]QASREFIAWLIAYKITE (SEQ ID NO 147);

H[Aib]EGTFSSELATILDEQAARDFIAWLIAHKITDkkkkkk([17-carboxy-Heptadecanoyl]-isoGlu)] (SEQ ID NO 148);

H[Aib]EGTFTSELATILDEQAARDFIAWLIAHKITDkkkkkk([17-carboxy-Heptadecanoyl]-isoGlu)] (SEQ ID NO 149);

H[AIb]EGSFTSELATILDEQAARDFIAWLIEHKITDkkkkkk([17-carboxy-Heptadecanoyl]-isoGlu)] (SEQ ID NO 150);

H[Aib]EGTFTSELATILD[K([19-Carboxy-nonadecanoyl]-isoGlu)]QAARDFIAWLIQHKITD (SEQ ID NO 151);

H[Aib]EGSFTSE[K([19-carboxy-nonadecanoyl]iso-Glu-Peg3-Peg3)]ATILDEQAARDFIAWLIEHKITD (SEQ ID NO 152);

H[Aib]EGSFTSELATILD[K([19-carboxy-nonadecanoyl]iso-Glu-Peg3-Peg3)]KAARDFIAWLIEHKITD (SEQ ID NO 153);

H[Aib]EGSFTSELATILEG[K([19-carboxy-nonadecanoyl]iso-Glu-Peg3-Peg3)]AARDFIAWLIEHKITD (SEQ ID NO 154);

H[Aib]EGSFTSELATILDEQAA[K([19-carboxy-nonadecanoyl]iso-Glu-Peg3-Peg3)]DFIAWLIEHKITD (SEQ ID NO

155);

H[Aib]EGTFTSELATILDEQAA[K([19-carboxy-nonadecanoyl]iso-Glu-Peg3-Peg3)]DFIAWLIEHKITD (SEQ ID NO 156);

H[Aib]EGTFSSELATILD[K([17-Carboxy-heptadecanoyl]-isoGlu-KEK-Peg3)]QAARDFIAWLIQHKITD (SEQ ID NO 157);

H[Aib]EGTFSSELATILD[K([19-Carboxy-nonadecanoyl]-isoGlu-KEK-Peg3)]QAARDFIAWLIQHKITD (SEQ ID NO 158);

H[Aib]EGTFSSELATILD[K([17-Carboxy-heptadecanoyl]-isoGlu-KEK-Peg3)]QAARDFIAWLIEHKITD (SEQ ID NO 159);

H[Aib]EGTFSSELATILD[K([19-Carboxy-nonadecanoyl]-isoGlu-KEK-Peg3)]QAARDFIAWLIEHKITD (SEQ ID NO 160);

H[Aib]EGTFTSELATILD[K([19-Carboxy-nonadecanoyl]-isoGlu-KEK)]QAARDFIAWLIQHKITD (SEQ ID NO 161);

H[Aib]EGTFTSELATILD[K([19-Carboxy-nonadecanoyl]-isoGlu-KEK-Peg3)]QAARDFIAWLIQHKITD (SEQ ID NO 162);

H[Aib]EGSFTSE[K([19-Carboxy-nonadecanoyl]-isoGlu-KEK-Peg3)]ATILDEQAARDFIAWLIEHKITD (SEQ ID NO 163);

H[Aib]EGTFTSE[K([19-Carboxy-nonadecanoyl]-isoGlu-KEK-Peg3)]ATILDEQAARDFIAWLIEHKITD (SEQ ID NO 164);

H[Aib]EGSFTSE[K([19-carboxy-nonadecanoyl]iso-Glu-KEK-Peg3-Peg3)]ATILDEQAARDFIAWLIEHKITD (SEQ ID NO 165);

H[Aib]EGTFTSELATILD[K([19-Carboxy-nonadecanoyl]-isoGlu-KEK-Peg3)]QAARDFIAWLIEHKITD (SEQ ID NO 166);

H[Aib]EGSFTSELATILD[K([19-Carboxy-nonadecanoyl]-isoGlu-KEK-Peg3)]QAARDFIAWLIEHKITD (SEQ ID NO 167);

H[Aib]EGSFTSELATILD[K([19-Carboxy-nonadecanoyl]-isoGlu-KEK-Peg3)]QAARDFIAWLIAHKITD (SEQ ID NO 168);

H[Aib]EGSFTSELATILD[K([19-Carboxy-nonadecanoyl]-isoGlu-KEK-Peg3)]KAARDFIAWLIEHKITD (SEQ ID NO 169);

H[Aib]EGSFTSELATILD[K([19-carboxy-nonadecanoyl]iso-Glu-KEK-Peg3-Peg3)]QAARDFIAWLIEHKITD (SEQ ID NO 170);

H[Aib]EGSFTSELATILEG[K([19-Carboxy-nonadecanoyl]-isoGlu-KEK-Peg3)]AARDFIAWLIEHKITD (SEQ ID NO 171);

H[Aib]EGSFTSELATILDA[K([19-Carboxy-nonadecanoyl]-isoGlu-KEK-Peg3)]AARDFIAWLIEHKITD (SEQ ID NO 172);

H[Aib]EGSFTSELATILDA[K([19-carboxy-nonadecanoyl]iso-Glu-KEK-Peg3-Peg3)]AARDFIAWLIEHKITD (SEQ ID NO 173);

H[Aib]EGSFTSELATILDEQAA[K([19-Carboxy-nonadecanoyl]-isoGlu-KEK-Peg3)]DFIAWLIEHKITD (SEQ ID NO 174);

H[Aib]EGTFTSELATILDEQAA[K([19-Carboxy-nonadecanoyl]-isoGlu-KEK-Peg3)]DFIAWLIEHKITD (SEQ ID NO 175);

H[Aib]EGSFTSELATILDEQAA[K([19-carboxy-nonadecanoyl]iso-Glu-KEK-Peg3-Peg3)]DFIAWLIEHKITD (SEQ ID NO 176);

H[Aib]EGTFTSELATILDEQAA[K([19-carboxy-nonadecanoyl]iso-Glu-KEK-Peg3-Peg3)]DFIAWLIEHKITD (SEQ ID NO 177); or

H[Aib]EGSFTSELATILDAKAA[K([19-Carboxy-nonadecanoyl]-isoGlu-KEK-Peg3)]DFIAWLIEHKITD (SEQ ID NO 178).

In preferred embodiments, peptide X* or the peptide X*-U has the sequence H[Aib]EGSFTSELATILD[K([17-carboxy-heptadecanoyl]-isoGlu)]QAARDFIAWLIQHKITD (SEQ ID NO 128).

The dual agonist may be:

Hy-H[Aib]EGTFSSELATILD[K([17-carboxy-heptadecanoyl]-isoGlu)]EAARDFIAWLIEHKITD-OH (Compound 1);

Hy-H[Aib]EGSFTSELATILD[K([17-carboxy-heptadecanoyl]-isoGlu)]EAARDFIAWLIEHKITD-OH (Compound 2);

Hy-H[Aib]EGTFTSELATILD[K([17-carboxy-heptadecanoyl]-isoGlu)]EAARDFIAWLIEHKITD-OH (Compound 3);

Hy-H[Aib]EGTFSSELATILD[K([17-carboxy-heptadecanoyl]-isoGlu)]KAARDFIAWLIEHKITD-OH (Compound 4);

Hy-H[Aib]EGSFTSELATILD[K([17-carboxy-heptadecanoyl]-isoGlu)]KAARDFIAWLIEHKITD-OH (Compound 5);

Hy-H[Aib]EGTFTSELATILD[K([17-carboxy-heptadecanoyl]-isoGlu)]KAARDFIAWLIEHKITD-OH (Compound 6);

Hy-H[Aib]EGTFSSELATILDG[K([17-carboxy-heptadecanoyl]-isoGlu)]AARDFIAWLIEHKITD-OH (Compound 7);

Hy-H[Aib]EGSFTSELATILDG[K([17-carboxy-heptadecanoyl]-isoGlu)]AARDFIAWLIEHKITD-OH (Compound 8);

Hy-H[Aib]EGTFTSELATILDG[K([17-carboxy-heptadecanoyl]-isoGlu)]AARDFIAWLIEHKITD-OH (Compound 9);

Hy-H[Aib]EGTFSSELATILD[K([17-carboxy-heptadecanoyl]-isoGlu)]LAARDFIAWLIEHKITD-OH (Compound 10);

Hy-H[Aib]EGSFTSELATILD[K([17-carboxy-heptadecanoyl]-isoGlu)]LAARDFIAWLIEHKITD-OH (Compound 11);

Hy-H[Aib]EGTFTSELATILD[K([17-carboxy-heptadecanoyl]-isoGlu)]LAARDFIAWLIEHKITD-OH (Compound 12);

Hy-H[Aib]EGTFSSELATILD[K([17-carboxy-heptadecanoyl]-isoGlu)]LAARDFIAWLIAHKITD-OH (Compound 13);

Hy-H[Aib]EGSFTSELATILD[K([17-carboxy-heptadecanoyl]-isoGlu)]LAARDFIAWLIAHKITD-OH (Compound 14);

Hy-H[Aib]EGTFTSELATILD[K([17-carboxy-heptadecanoyl]-isoGlu)]LAARDFIAWLIAHKITD-OH (Compound 15);

Hy-H[Aib]EGTFTSELATI LD[K([17-carboxy-heptadecanoyl]-isoGlu)]EAARLFIAWLIEHKITD-OH (Compound 16);

Hy-H[Aib]EGTFSSELATILD[K([17-carboxy-heptadecanoyl]-isoGlu)]QAARDFIAWLIQHKITD-OH (Compound 17);

Hy-H[Aib]EGSFTSELATILD[K([17-carboxy-heptadecanoyl]-isoGlu)]QAARDFIAWLIQHKITD-OH (Compound 18);

Hy-H[Aib]EGTFTSELATILD[K([17-carboxy-heptadecanoyl]-isoGlu)]QAARDFIAWLIQHKITD-OH (Compound 19);

Hy-H[Aib]EGTFSSELATILD[K([17-carboxy-heptadecanoyl]-isoGlu)]QAARDFIAWLIEHKITD-OH (Compound 20);

Hy-H[Aib]EGTFSSELATILD[K([17-carboxy-heptadecanoyl]-isoGlu)]QAARDFIAWLIAHKITD-OH (Compound 21);

Hy-H[Aib]EGSFTSELATILD[K([17-carboxy-heptadecanoyl]-isoGlu)]QAARDFIAWLIAHKITD-OH (Compound 22);

Hy-H[Aib]EGTFTSELATILD[K([17-carboxy-heptadecanoyl]-isoGlu)]QAARDFIAWLIAHKITD-OH (Compound 23);

Hy-H[Aib]EGSFTSELATILD[K([17-carboxy-heptadecanoyl]-isoGlu)]QAARDFIAWLIEHKITD-OH (Compound 24);

Hy-H[Aib]EGTFTSELATILD[K([17-carboxy-heptadecanoyl]-isoGlu)]QAARDFIAWLIEHKITD-OH (Compound 25);

Hy-H[Aib]EGSFTSELATILD[K([17-carboxy-heptadecanoyl]-isoGlu)]QAARDFIAWLIHHKITD-OH (Compound 26);

Hy-H[Aib]EGSFTSELATILD[K([17-carboxy-heptadecanoyl]-isoGlu)]QAARDFIAWLIYHKITD-OH (Compound 27);

Hy-H[Aib]EGSFTSELATILD[K([17-carboxy-heptadecanoyl]-isoGlu)]QAARDFIAWLILHKITD-OH (Compound 28);

Hy-H[Aib]EGSFTSELATILD[K([17-carboxy-heptadecanoyl]-isoGlu)]QAARDFIAWLIKHKITD-OH (Compound 29);

Hy-H[Aib]EGSFTSELATILD[K([17-carboxy-heptadecanoyl]-isoGlu)]QAARDFIAWLIRHKITD-OH (Compound 30);

Hy-H[Aib]EGSFTSELATILD[K([17-carboxy-heptadecanoyl]-isoGlu)]QAARDFIAWLISHKITD-OH (Compound 31).

Hy-H[Aib]EGSFTSELATILD[K([Hexadecanoyl]-βAla)]QAARDFIAWLQQHKITD-OH (Compound 32);

Hy-H[Aib]EGSFTSELATILD[K([17-carboxy-heptadecanoyl]iso-Glu-Peg3)]QAARDFIAWLYQHKITD-OH (Compound 33);

Hy-H[Aib]EGSFTSELATILD[K([19-carboxy-nonadecanoyl]iso-Glu-Peg3-Peg3)]QAARDFIAWLKQHKITD-OH (Compound 34);

Hy-H[Aib]EGSFTSELATILD[K([19-carboxy-nonadecanoyl]iso-Lys-Peg3-Peg3-Peg3)]QAARDFIAWLIQQKITD-OH (Compound 35);

Hy-H[Aib]EGSFTSELATILD[K(Octadecanoyl)]QAARDFIAWLIQYKITD-OH (Compound 36);

Hy-H[Aib]EGTFSSELSTILE[K(Hexadecanoyl-isoGlu)]QASREFIAWLIAYKITE-OH (Compound 37);

Hy-H[Aib]EGTFSSELATILDEQAARDFIAWLIAHKITDkkkkkk([17-carboxy-Heptadecanoyl]-isoGlu)]-[NH2] (Compound 38);

Hy-H[Aib]EGTFTSELATILDEQAARDFIAWLIAHKITDkkkkkk([17-carboxy-Heptadecanoyl]-isoGlu)]-[NH2] (Compound 39);

Hy-H[Aib]EGSFTSELATILDEQAARDFIAWLIEHKITDkkkkkk([17-carboxy-Heptadecanoyl]-isoGlu)]-[NH2] (Compound 40);

Hy-H[Aib]EGTFTSELATILD[K([19-Carboxy-nonadecanoyl]-isoGlu)]QAARDFIAWLIQHKITD-OH (Compound 41);

Hy-H[Aib]EGSFTSE[K([19-carboxy-nonadecanoyl]iso-Glu-Peg3-Peg3)]ATILDEQAARDFIAWLIEHKITD-OH (Compound 42);

Hy-H[Aib]EGSFTSELATILD[K([19-carboxy-nonadecanoyl]iso-Glu-Peg3-Peg3)]KAARDFIAWLIEHKITD-OH (Compound 43);

Hy-H[Aib]EGSFTSELATILEG[K([19-carboxy-nonadecanoyl]iso-Glu-Peg3-Peg3)]AARDFIAWLIEHKITD-OH (Compound 44);

Hy-H[Aib]EGSFTSELATILDEQAA[K([19-carboxy-nonadecanoyl]iso-Glu-Peg3-Peg3)]DFIAWLIEHKITD-OH (Compound 45);

Hy-H[Aib]EGTFTSELATILDEQAA[K([19-carboxy-nonadecanoyl]iso-Glu-Peg3-Peg3)]DFIAWLIEHKITD-OH (Compound 46).

Hy-H[Aib]EGTFSSELATILD[K([17-Carboxy-heptadecanoyl]-isoGlu-KEK-Peg3)]QAARDFIAWLIQHKITD-OH (Compound 47);

Hy-H[Aib]EGTFSSELATILD[K([19-Carboxy-nonadecanoyl]-isoGlu-KEK-Peg3)]QAARDFIAWLIQHKITD-OH (Compound 48);

Hy-H[Aib]EGTFSSELATILD[K([17-Carboxy-heptadecanoyl]-isoGlu-KEK-Peg3)]QAARDFIAWLIEHKITD-OH (Compound 49);

Hy-H[Aib]EGTFSSELATILD[K([19-Carboxy-nonadecanoyl]-isoGlu-KEK-Peg3)]QAARDFIAWLIEHKITD-OH (Compound 50);

Hy-H[Aib]EGTFTSELATILD[K([19-Carboxy-nonadecanoyl]-isoGlu-KEK)]QAARDFIAWLIQHKITD-OH (Compound 51);

Hy-H[Aib]EGTFTSELATILD[K([19-Carboxy-nonadecanoyl]-isoGlu-KEK-Peg3)]QAARDFIAWLIQHKITD-OH (Compound 52);

Hy-H[Aib]EGSFTSE[K([19-Carboxy-nonadecanoyl]-isoGlu-KEK-Peg3)]ATILDEQAARDFIAWLIEHKITD-OH (Compound 53);

Hy-H[Aib]EGTFTSE[K([19-Carboxy-nonadecanoyl]-isoGlu-KEK-Peg3)]ATILDEQAARDFIAWLIEHKITD-OH (Compound 54);

Hy-H[Aib]EGSFTSE[K([19-carboxy-nonadecanoyl]iso-Glu-KEK-Peg3-Peg3)]ATILDEQAARDFIAWLIEHKITD-OH (Compound 55);

Hy-H[Aib]EGTFTSELATILD[K([19-Carboxy-nonadecanoyl]-isoGlu-KEK-Peg3)]QAARDFIAWLIEHKITD-OH (Compound 56);

Hy-H[Aib]EGSFTSELATILD[K([19-Carboxy-nonadecanoyl]-isoGlu-KEK-Peg3)]QAARDFIAWLIEHKITD-OH (Compound 57);

Hy-H[Aib]EGSFTSELATILD[K([19-Carboxy-nonadecanoyl]-isoGlu-KEK-Peg3)]QAARDFIAWLIAHKITD-OH (Compound 58);

Hy-H[Aib]EGSFTSELATILD[K([19-Carboxy-nonadecanoyl]-isoGlu-KEK-Peg3)]KAARDFIAWLIEHKITD-OH (Compound 59);

Hy-H[Aib]EGSFTSELATILD[K([19-carboxy-nonadecanoyl]iso-Glu-KEK-Peg3-Peg3)]QAARDFIAWLIEHKITD-OH (Compound 60);

Hy-H[Aib]EGSFTSELATILEG[K([19-Carboxy-nonadecanoyl]-isoGlu-KEK-Peg3)]AARDFIAWLIEHKITD-OH (Compound 61);

Hy-H[Aib]EGSFTSELATILDA[K([19-Carboxy-nonadecanoyl]-isoGlu-KEK-Peg3)]AARDFIAWLIEHKITD-OH (Compound 62);

Hy-H[Aib]EGSFTSELATILDA[K([19-carboxy-nonadecanoyl]iso-Glu-KEK-Peg3-Peg3)]AARDFIAWLIEHKITD-OH (Compound 63);

Hy-H[Aib]EGSFTSELATILDEQAA[K([19-Carboxy-nonadecanoyl]-isoGlu-KEK-Peg3)]DFIAWLIEHKITD-OH (Compound 64);

Hy-H[Aib]EGTFTSELATILDEQAA[K([19-Carboxy-nonadecanoyl]-isoGlu-KEK-Peg3)]DFIAWLIEHKITD-OH (Compound 65);

Hy-H[Aib]EGSFTSELATILDEQAA[K([19-carboxy-nonadecanoyl]iso-Glu-KEK-Peg3-Peg3)]DFIAWLIEHKITD-OH (Compound 66);

Hy-H[Aib]EGTFTSELATILDEQAA[K([19-carboxy-nonadecanoyl]iso-Glu-KEK-Peg3-Peg3)]DFIAWLIEHKITD-OH (Compound 67); or

Hy-H[Aib]EGSFTSELATILDAKAA[K([19-Carboxy-nonadecanoyl]-isoGlu-KEK-Peg3)]DFIAWLIEHKITD-OH (Compound 68).

The dual agonist of the invention is Hy-H[Aib]EGSFTSELATILD[K([17-carboxy-heptadecanoyl]-isoGlu)]QAARDFIAWLIQHKITD-OH (Compound 18).

A GLP-1/GLP-2 dual agonist as described herein may be a compound represented by the formula: R1-X*-U-R2, wherein:

R1 is hydrogen (Hy), C1-4 alkyl (e.g. methyl), acetyl, formyl, benzoyl or trifluoroacetyl;

R2 is NH2 or OH;

X* is a peptide of formula I (SEQ ID NO 1):
H-X2-EG-X5-F-X7-X8-E-X10-X11-TI L-X15-X16-X17-A-X19-X20-X21-FI-X24-WL-X27-X28-X29-KIT-X33 (I)

wherein

X2 is Aib or G; X5 is S or T; X7 is S or T; X8 is S, E or D; X10 is L, M or V; X11 is A, N or S; X15 is D or E; X16 is E, A or G; X17 is Q, E, L or K; X19 is A, V or S; X20 is R or K; X21 is D, L or E; X24 is A, N or S; X27 is I, Y, Q, H or K; X28 is A, E, H, Y, L, K, Q, R or S; X29 is H, Y, K or Q; X33 is D or E;

U is absent or a sequence of 1-15 residues, each independently selected from K and k; and

wherein at least one of X5 and X7 is T;

or a pharmaceutically acceptable salt or solvate thereof.

The invention provides a composition comprising a GLP-1/GLP-2 dual agonist for use in a method for the treatment and/or prevention of diarrhea in a patient having undergone surgical resection of the bowel, wherein the GLP-1/GLP-2 dual agonist is Hy-H[Aib]EGSFTSELATILD[K([17-carboxy-heptadecanoyl]-isoGlu)]QAARDFIAWLIQHKITD-OH (Compound 18).

The dual agonist according to the invention may be in the form of a pharmaceutically acceptable salt or solvate, such as a pharmaceutically acceptable acid addition salt.

The dual agonist may be in the form of a composition, which, for example, may comprise the dual agonist, or a pharmaceutically acceptable salt or solvate thereof, together with a carrier, excipient or vehicle. The carrier may be a pharmaceutically acceptable carrier.

The composition may be a pharmaceutical composition. The pharmaceutical composition may be formulated as a liquid suitable for administration by injection or infusion. It may be formulated to achieve slow release of the dual agonist.

The dual agonist may be administered at a dose of about 0.1 pmol/kg to 500μmol/kg body weight.

**Biological activity of GLP-1/GLP-2 dual agonist**

The terms "GLP-1/GLP-2 dual agonist" and "GLP-1/GLP-2 dual receptor agonist" are used interchangeably herein and have the same meaning. The dual agonists/dual receptor agonists have agonist activity at both of the GLP-1 and GLP-2 receptors, e.g. the human GLP-1 and GLP-2 receptors.

The dual agonist has at least one GLP-1 and at least one GLP-2 biological activity.

Exemplary GLP-1 physiological activities include reducing rate of intestinal transit, reducing rate of gastric emptying, reducing appetite, food intake or body weight, and improving glucose control and glucose tolerance. Exemplary GLP-2 physiological activities include causing an increase in intestinal mass (e.g. of small intestine or colon), intestinal repair, and improving intestinal barrier function (i.e. reducing permeability of the intestine). These parameters can be

assessed in *in vivo* assays in which the mass and the permeability of the intestine, or a portion thereof, is determined after a test animal has been treated with a dual agonist.

The dual agonists have agonist activity at the GLP-1 and GLP-2 receptors, e.g. the human GLP-1 and GLP-2 receptors. $EC_{50}$ values for *in vitro* receptor agonist activity may be used as a numerical measure of agonist potency at a given receptor. An $EC_{50}$ value is a measure of the concentration (e.g. mol/L) of a compound required to achieve half of that compound's maximal activity in a particular assay. A compound having a numerical $EC_{50}$ at a particular receptor which is lower than the $EC_{50}$ of a reference compound in the same assay may be considered to have higher potency at that receptor than the reference compound.

GLP-1 activity

In some embodiments, the dual agonist has an $EC_{50}$ at the GLP-1 receptor (e.g. the human GLP-1 receptor) which is below 2.0 nM, below 1.5 nM, below1.0 nM, below 0.9 nM, below 0.8 nM, below 0.7 nM, below 0.6 nM, below 0.5 nM, below 0.4 nM, below 0.3 nM, below 0.2 nM, below 0.1 nM, below 0.09 nM, below 0.08 nM, below 0.07 nM, below 0.06 nM, below 0.05 nM, below 0.04 nM, e.g. when assessed using the GLP-1 receptor potency assay described in Example 2 of WO2018/104561.

In some embodiments, the dual agonist has an $EC_{50}$ at the GLP-1 receptor which is between 0.005 and 2.5 nM, between 0.01 nM and 2.5 nM, between 0.025 and 2.5 nM, between 0.005 and 2.0 nM, between 0.01 nM and 2.0 nM, between 0.025 and 2.0 nM, between 0.005 and 1.5 nM, between 0.01 nM and 1.5 nM, between 0.025 and 1.5 nM, between 0.005 and 1.0 nM, between 0.01 nM and 1.0 nM, between 0.025 and 1.0 nM, between 0.005 and 0.5 nM, between 0.01 nM and 0.5 nM, between 0.025 and 0.5 nM, between 0.005 and 0.25 nM, between 0.01 nM and 0.25 nM, between 0.025 and 0.25 nM, e.g. when assessed using the GLP-1 receptor potency assay described in Example 2 of WO2018/104561.

An alternative measure of GLP-1 agonist activity may be derived by comparing the potency of a dual agonist with the potency of a known (or reference) GLP-1 agonist when both are measured in the same assay. Thus, the relative potency at the GLP-1 receptor may be defined as:

$$[EC_{50}(\text{reference agonist})] / [EC_{50}(\text{dual agonist})].$$

Thus a value of 1 indicates that the dual agonist and reference agonist have equal potency, a value of >1 indicates that the dual agonist has higher potency (i.e. lower $EC_{50}$) than the reference agonist, and a value of <1 indicates that the dual agonist has lower potency (i.e. higher $EC_{50}$) than the reference agonist.

The reference GLP-1 agonist may, for example, be human GLP-1(7-37), liraglutide (NN2211; Victoza), or Exendin-4, but is preferably liraglutide.

Typically the relative potency will be between 0.001 and 100, e.g.

between 0.001 and 10, between 0.001 and 5, between 0.001 and 1, between 0.001 and 0.5, between 0.001 and 0.1, between 0.001 and 0.05, or between 0.001 and 0.01;

between 0.01 and 10, between 0.01 and 5, between 0.01 and 1, between 0.01 and 0.5, between 0.01 and 0.1, or between 0.01 and 0.05;

between 0.05 and 10, between 0.05 and 5, between 0.05 and 1, between 0.05 and 0.5, or between 0.05 and 0.1;

between 0.1 and 10, between 0.1 and 5, between 0.1 and 1, or between 0.1 and 0.5;

between 0.5 and 10, between 0.5 and 5, or between 0.5 and 1;

between 1 and 10, or between 1 and 5;

or between 5 and 10.

The dual agonist according to the invention may have higher potency at the GLP-1 receptor (e.g. the human GLP-1 receptor) than wild type human GLP-2 (hGLP-2 (1-33)) or [Gly2]-hGLP-2 (1-33) (i.e. human GLP-2 having glycine at position 2, also known as teduglutide). Thus, the relative potency of the dual agonists at the GLP-1 receptor compared to hGLP-2 (1-33) or teduglutide is greater than 1, typically greater than 5 or greater than 10, and may be up to 100, up to 500, or even higher.

GLP-2 activity

In some embodiments, the dual agonist has an $EC_{50}$ at the GLP-2 receptor (e.g. the human GLP-2 receptor) which is below 2.0 nM, below 1.5 nM, below1.0 nM, below 0.9 nM, below 0.8 nM, below 0.7 nM, below 0.6 nM, below 0.5 nM, below 0.4 nM, below 0.3 nM, below 0.2 nM, below 0.1 nM, below 0.09 nM, below 0.08 nM, below 0.07 nM, below 0.06 nM, below 0.05 nM, below 0.04 nM, below 0.03 nM, below 0.02 nM, or below 0.01 nM, e.g. when assessed using the GLP-1 receptor potency assay described in Example 2 of WO2018/104561.

In some embodiments, the dual agonist has an $EC_{50}$ at the GLP-2 receptor which is between 0.005 and 2.0 nM, between 0.01 nM and 2.0 nM, between 0.025 and 2.0 nM, between 0.005 and 1.5 nM, between 0.01 nM and 1.5 nM, between 0.025 and 1.5 nM, between 0.005 and 1.0 nM, between 0.01 nM and 1.0 nM, between 0.025 and 1.0 nM, between 0.005 and 0.5 nM, between 0.01 nM and 0.5 nM, between 0.025 and 0.5 nM, between 0.005 and 0.25 nM, between 0.01 nM and 0.25 nM, between 0.025 and 0.25 nM, e.g. when assessed using the GLP-2 receptor potency assay described in Example 2 of WO2018/104561.

An alternative measure of GLP-2 agonist activity may be derived by comparing the potency of a dual agonist with the potency of a known (or reference) GLP-2 agonist when both are measured in the same assay. Thus the relative potency at the GLP-2 receptor may be defined as:

$$[EC_{50}(\text{reference agonist})] / [EC_{50}(\text{dual agonist})].$$

Thus a value of 1 indicates that the dual agonist and reference agonist have equal potency, a value of >1 indicates that the dual agonist has higher potency (i.e. lower $EC_{50}$) than the reference agonist, and a value of <1 indicates that the dual agonist has lower potency (i.e. higher $EC_{50}$) than the reference agonist.

The reference GLP-2 agonist may, for example, be human GLP-2(1-33) or teduglutide ([Gly2]-hGLP-2 (1-33)), but is preferably teduglutide. Typically the relative potency will be between 0.001 and 100, e.g.

between 0.001 and 10, between 0.001 and 5, between 0.001 and 1, between 0.001 and 0.5, between 0.001 and 0.1, between 0.001 and 0.05, or between 0.001 and 0.01;

between 0.01 and 10, between 0.01 and 5, between 0.01 and 1, between 0.01 and 0.5, between 0.01 and 0.1, or between 0.01 and 0.05;

between 0.05 and 10, between 0.05 and 5, between 0.05 and 1, between 0.05 and 0.5, or between 0.05 and 0.1;

between 0.1 and 10, between 0.1 and 5, between 0.1 and 1, or between 0.1 and 0.5;

between 0.5 and 10, between 0.5 and 5, or between 0.5 and 1;

between 1 and 10, or between 1 and 5;

or between 5 and 10.

The dual agonists according to the invention may have higher potency at the GLP-2 receptor (e.g. the human GLP-2 receptor) than human GLP-1(7-37), liraglutide (NN2211; Victoza), or Exendin-4. Thus, the relative potency of the dual agonists at the GLP-2 receptor compared to human GLP-1(7-37), liraglutide (NN2211; Victoza), or Exendin-4 is greater than 1, typically greater than 5 or greater than 10, and may be up to 100, up to 500, or even higher (if the reference GLP-1 agonist even exerts detectable activity at the GLP-2 receptor).

It will be understood that the absolute potencies of the dual agonists at each receptor are much less important than the balance between the GLP-1 and GLP-2 agonist activities. Thus, it is perfectly acceptable for the absolute GLP-1 or GLP-2 potency to be lower than that of known agonists at those receptors, as long as the dual agonist compound exerts acceptable relative levels of potency at both receptors. Any apparent deficiency in absolute potency can be compensated by an increased dose if required.

**Synthesis of GLP-1/GLP-2 dual agonist**

The dual agonists according to the invention may be synthesized according to the methods set out in International publication numbers WO2018/104560 and WO2018/104561.

Dual agonists may be synthesised by means of solid-phase or liquid-phase peptide synthesis methodology. In

this context, reference may be made to WO98/11125 and, among many others, Fields, G.B. et al., 2002, "Principles and practice of solid-phase peptide synthesis". In: Synthetic Peptides (2nd Edition), and the Example 1 of WO2018/104561.

**Pharmaceutical Compositions and Administration**

The dual agonist used in the present invention may be in the form of a composition comprising a dual agonist, or a pharmaceutically acceptable salt or solvate thereof, together with a carrier. In one embodiment of the invention, the composition is a pharmaceutical composition and the carrier is a pharmaceutically acceptable carrier. The pharmaceutical composition comprising a dual agonist according to the invention, or a salt or solvate thereof, may be together with a carrier, excipient or vehicle. Accordingly, the dual agonist, or salts or solvates thereof, especially pharmaceutically acceptable salts or solvates thereof, may be formulated as compositions or pharmaceutical compositions prepared for storage or administration, and which comprise a therapeutically effective amount of a dual agonist, or a salt or solvate thereof. An example of formulating a GLP-1/GLP-2 dual agonist can be found in international publication WO2020249782A1.

Suitable salts formed with bases include metal salts, such as alkali metal or alkaline earth metal salts, for example sodium, potassium or magnesium salts; ammonia salts and organic amine salts, such as those formed with morpholine, thiomorpholine, piperidine, pyrrolidine, a lower mono-, di- or tri-alkylamine (e.g., ethyl-tert-butyl-, diethyl-, diisopropyl-, triethyl-, tributyl- or dimethylpropylamine), or a lower mono-, di- or tri-(hydroxyalkyl)amine *(e.g.,* mono-, di- or triethanolamine). Internal salts may also be formed. Similarly, when a compound of the present invention contains a basic moiety, salts can be formed using organic or inorganic acids. For example, salts can be formed from the following acids: formic, acetic, propionic, butyric, valeric, caproic, oxalic, lactic, citric, tartaric, succinic, fumaric, maleic, malonic, mandelic, malic, phthalic, hydrochloric, hydrobromic, phosphoric, nitric, sulphuric, benzoic, carbonic, uric, methanesulphonic, naphthalenesulphonic, benzenesulphonic, toluenesulphonic, p-toluenesulphonic (i.e. 4-methylbenzene-sulphonic), camphorsulphonic, 2-aminoethanesulphonic, aminomethylphosphonic and trifluoromethanesulphonic acid (the latter also being denoted triflic acid), as well as other known pharmaceutically acceptable acids. Amino acid addition salts can also be formed with amino acids, such as lysine, glycine, or phenylalanine.

In one embodiment is used a pharmaceutical composition wherein the dual agonist is in the form of a pharmaceutically acceptable acid addition salt.

As will be apparent to one skilled in the medical art, a "therapeutically effective amount" of a dual agonist or pharmaceutical composition thereof according to the present invention will vary depending upon, inter alia, the age, weight and/or gender of the subject (patient) to be treated. Other factors that may be of relevance include the physical characteristics of the specific patient under consideration, the patient's diet, the nature of any concurrent medication, the particular compound(s) employed, the particular mode of administration, the desired pharmacological effect(s) and the particular therapeutic indication. Because these factors and their relationship in determining this amount are well known in the medical arts, the determination of therapeutically effective dosage levels, the amount necessary to achieve the desired result of treating and/or preventing and/or remedying malabsorption and/or low-grade inflammation described herein, as well as other medical indications disclosed herein, will be within the ambit of the skilled person.

As used herein, the term "a therapeutically effective amount" refers to an amount which reduces symptoms of a given condition or pathology, and preferably which normalizes physiological responses in an individual with that condition or pathology. Reduction of symptoms or normalization of physiological responses can be determined using methods routine in the art and may vary with a given condition or pathology. In one aspect, a therapeutically effective amount of one or more dual agonist, or pharmaceutical compositions thereof, is an amount which restores a measurable physiological parameter to substantially the same value (preferably to within 30%, more preferably to within 20%, and still more preferably to within 10% of the value) of the parameter in an individual without the condition or pathology in question.

**Dosing**

In one embodiment of the invention, administration of a dual agonist according to the present invention is commenced at lower dosage levels, with dosage levels being increased until the desired effect of preventing/treating the relevant medical indication is achieved. This would define a therapeutically effective amount. For the dual agonist according to the present invention, alone or as part of a pharmaceutical composition, such human doses of the active dual agonist may be between about 0.01 pmol/kg and 500 μmol/kg body weight, between about 0.1 pmol/kg and 300 μmol/kg body weight, between 0.01 pmol/kg and 100 μmol/kg body weight, between 0.1 pmol/kg and 50 μmol/kg body weight, between 1 pmol/kg and 10 μmol/kg body weight, between 5 pmol/kg and 5 μmol/kg body weight, between 10 pmol/kg and 1 μmol/kg body weight, between 50 pmol/kg and 0.1 μmol/kg body weight, between 100 pmol/kg and 0.01 μmol/kg body weight, between 0.001 μmol/kg and 0.5 μmol/kg body weight, between 0.05 μmol/kg and 0.1 μmol/kg body weight.

In one aspect the dose is in the range of about 50pmol/kg to 500 nmol/kg, for example about 60pmol/kg to 400 nmol/kg, about 70pmol/kg to 300 nmol/kg, about 80pmol/kg to 200 nmol/kg, about 90pmol/kg to 100 nmol/kg.

In one aspect the dose is in the nmol range, for example between 1 nmol/kg and 100nmol/kg, between 1nmol/kg and 90nmol/kg, between 1nmol/kg and 80nmol/kg, between 1nmol/kg and 70nmol/kg, between 1nmol/kg and 60nmol/kg, between 1 nmol/kg and 50nmol/kg, between 1 nmol/kg and 40nmol/kg, between 1 nmol/kg and 30nmol/kg, between 1nmol/kg and 20nmol/kg, or between 1nmol/kg and 10nmol/kg. The therapeutic dosing and regimen most appropriate for patient treatment will of course vary with the disease or condition to be treated, and according to the patient's weight and other parameters. Without wishing to be bound by any particular theory, it is expected that doses, in the pmol/kg or nmol/kg range, and shorter or longer duration or frequency of treatment may produce therapeutically useful results, such as a statistically significant increase particularly in small bowel mass. In some instances, the therapeutic regimen may include the administration of maintenance doses appropriate for preventing tissue regression that occurs following cessation of initial treatment. The dosage sizes and dosing regimen most appropriate for human use may be guided by the results obtained by the present invention, and may be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person.

In one aspect the dual agonist according to the invention is administered intravenously, in a continuous infusion.

In one aspect the patient is an adult. In one aspect the patient is a neonate. In one aspect the patient is a child.

**Bowel resection**

The present invention relates to the field of bowel resection.

A bowel resection is a surgery to remove any part of the bowel. This includes the small intestine, large intestine, or rectum. A bowel resection according to the invention may be performed as required for a particular patient.

The large intestine, also known as the large bowel, is the last part of the gastrointestinal tract and of the digestive system in vertebrates. Water is absorbed here and the remaining waste material is stored as faeces before being removed by defecation.

In humans, the large intestine begins in the right iliac region of the pelvis, just at or below the waist, where it is joined to the end of the small intestine at the cecum, via the ileocecal valve. It then continues as the colon ascending the abdomen, across the width of the abdominal cavity as the transverse colon, and then descending to the rectum and its endpoint at the anal canal. Overall, in humans, the large intestine is about 1.5 metres (5 ft) long, which is about one-fifth of the whole length of the gastrointestinal tract.

The small intestine or small bowel is an organ in the gastrointestinal tract where most of the end absorption of nutrients and minerals from food takes place. It lies between the stomach and large intestine, and receives bile and pancreatic juice through the pancreatic duct to aid in digestion.

The small intestine has three distinct regions - the duodenum, jejunum, and ileum. The duodenum, the shortest, is where preparation for absorption through small finger-like protrusions called villi begins. The jejunum is specialized for the absorption through its lining by enterocytes: small nutrient particles which have been previously digested by enzymes in the duodenum. The main function of the ileum is to absorb vitamin B12, bile salts, and whatever products of digestion were not absorbed by the jejunum.

Different types of bowel resections may be performed to remove different parts of the bowel. Each type of bowel resection is named based on what it removes.

A segmental small bowel resection removes part of the small intestine. Some of the mesentery (a fold of tissue that supports the small intestine) and lymph nodes in the area may also be removed.

A segmental small bowel resection is used to remove tumours in the lower part of the duodenum, in the jejunum or in the ileum if the cancer is only in these structures or if it has spread just beyond the small intestine.

A right hemicolectomy removes:

part of the ileum

the cecum

the ascending colon (the first part of the colon)

the hepatic flexure (the bend in the colon near the liver)

the first part of the transverse colon (the middle of the colon)

the appendix

A right hemicolectomy is used to remove tumours in the right colon, including the cecum and ascending colon. It may also be done to remove tumours of the appendix.
An extended right hemicolectomy removes all of the transverse colon. It may be done to remove tumours in the hepatic flexure or transverse colon.
A transverse colectomy removes the transverse colon. This surgery may be done to remove a tumour in the middle of the transverse colon when the cancer hasn't spread to any other parts of the colon. Sometimes an extended right hemicolectomy is done instead.
A left hemicolectomy removes:

part of the transverse colon

the splenic flexure (the bend in the colon near the spleen)

the descending colon

part of the sigmoid colon

A low anterior resection removes the sigmoid colon and part of the rectum. It is used to remove tumours in the middle or upper part of the rectum.
A proctocolectomy (also called a proctectomy) removes all of the rectum and part of the sigmoid colon. Coloanal anastomosis joins the remaining colon to the anus.
This surgery is used to remove tumours in the lower part of the rectum. Often a low anterior resection or an abdominoperineal resection is done instead.
An abdominoperineal resection removes the rectum, anus, anal sphincter and muscles around the anus. It is used to remove tumours that are close to the anus or have grown into the muscles around the anus.
A subtotal or total colectomy removes part or all of the colon. If most of the colon is removed, it is called a subtotal or partial colectomy. If all of the colon is removed, including the cecum and the appendix, it is called a total colectomy.
A subtotal or total colectomy is done:

when there is cancer on both the right and left sides of the colon

as a way to prevent colorectal cancer (called a prophylactic colectomy) for some people with familial adenomatous polyposis (FAP) or Lynch syndrome (also called hereditary non-polyposis colon cancer or HNPCC)

to remove a colon that is damaged by an inflammatory bowel disease

Depending on the type of colectomy done, the surgeon may also need to do a colostomy or an ileostomy.
In one aspect of the invention, about 0.5, 0.6, 0.7, 0.8, 0.9, 1.0, 2.0, 3.0, 4.0, 5.0, 6.0, 7.0, 8.0, 9.0, 10.0, 11.0, 12.0, 13.0, 14.0, 15.0, 16.0, 17.0, 18.0, 19.0, or 20.0% or more, such as about 30, 40, 50, 60, 70, 80 or 90% of the bowel of said patient is removed.
The length of bowel removed may depend on the total length of the bowel of the patient, for example about 5cm or more may be removed. In one aspect at least about 30cm of bowel is retained.

**Short bowel syndrome (SBS)**

In one aspect of the invention the patient has short bowel syndrome (SBS).
In preferred embodiments, the invention provides a composition comprising a GLP-1/GLP-2 dual agonist for use in a method for the treatment and/or prevention of diarrhea in a patient having SBS.
SBS usually results from surgical resection of some or most of the small intestine for conditions such as Crohn's disease, mesenteric infarction, volvulus, trauma, congenital anomalies, and multiple strictures due to adhesions or radiation.
SBS patients suffer from malabsorption that may lead to malnutrition, dehydration and weight loss. Some patients can maintain their protein and energy balance through hyperphagia; more rarely they can sustain fluid and electrolyte requirements to become independent from parenteral fluid.
Short bowel syndrome (SBS) is anatomically defined as that symptom complex which occurs in adults who have less than 200 centimeters of combined jejunum-ileum following small bowel resection. As the intestinal length in children is linked to the state of growth, a definition of a short bowel in absolute terms has not been devised. The need for intravenous supplementation or a residual short bowel length of less than 25% expected for gestational age are suggested definitions

of a short bowel in children

The syndrome is characterized by weight loss, dehydration, malnutrition, and malabsorption of macro- and micronutrients. Note that some patients who have had extensive bowel resections leaving them with more than 200 centimeters of small intestine can develop symptoms indistinguishable from those who fulfill the technical criteria for SBS. This situation occurs when the remaining bowel is diseased as, for example, in patients with Crohn's disease or radiation enteritis. These latter patients are generally managed like the standard SBS patient.

In one aspect of the invention, the patient has SBS and stoma.

**Stoma**

An artificial stoma is an opening in the body as the result of a surgical procedure. An artificial stoma on the abdomen can be connected to either the digestive or urinary system to allow waste (urine or faeces) to be diverted out of the body to relieve gastrointestinal disease symptoms. The stoma is created at an end of the intestine, which is brought to the surface of the abdomen to form the stoma (opening).

A stoma which is formed after the large bowel or colon is known as a colostomy. A stoma where a part of the small bowel (ileum) forms the opening is known as ileostomy.

The term "stomal output" means the amount of feces leaving the stoma.

**Definitions**

Unless specified otherwise, the following definitions are provided for specific terms which are used in the present written description.

Throughout this specification, the word "comprise", and grammatical variants thereof, such as "comprises" or "comprising", will be understood to imply the inclusion of a stated integer or component, or group of integers or components, but not the exclusion of any other integer or component, or group of integers or components.

The singular forms "a," "an," and "the" include the plurals unless the context clearly dictates otherwise.

The term "including" is used to mean "including but not limited to". "Including" and "including but not limited to" may be used interchangeably.

The terms "patient", "subject" and "individual" may be used interchangeably and refer to either a human or a non-human animal. These terms include mammals such as humans, primates, livestock animals (e.g., bovines and porcines), companion animals (e.g., canines and felines) and rodents (e.g., mice and rats). In one aspect the patient is a human.

As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein.

The term "solvate" in the context of the present invention refers to a complex of defined stoichiometry formed between a solute *(in casu,* a peptide or pharmaceutically acceptable salt thereof according to the invention) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

The term "agonist" as employed in the context of the invention refers to a substance (ligand) that activates the receptor type in question.

Throughout the present description and claims the conventional three-letter and one-letter codes for naturally occurring amino acids are used, i.e.

A (Ala), G (Gly), L (Leu), I (Ile), V (Val), F (Phe), W (Trp), S (Ser), T (Thr), Y (Tyr), N (Asn), Q (Gln), D (Asp), E (Glu), K (Lys), R (Arg), H (His), M (Met), C (Cys) and P (Pro);
as well as generally accepted three-letter codes for other $\alpha$-amino acids, such as sarcosine (Sar), norleucine (Nle), $\alpha$-aminoisobutyric acid (Aib), 2,3-diaminopropanoic acid (Dap), 2,4-diaminobutanoic acid (Dab) and 2,5-diaminopentanoic acid (ornithine; Orn). Such other $\alpha$-amino acids may be shown in square brackets "[ ]" (e.g. "[Aib]") when used in a general formula or sequence in the present specification, especially when the rest of the formula or sequence is shown using the single letter code. Unless otherwise specified, amino acid residues in peptides of the invention are of the L-configuration. However, D-configuration amino acids may be incorporated. In the present context, an amino acid code written with a small letter represents the D-configuration of said amino acid, e.g. "k" represents the D-configuration of lysine (K).

Among sequences disclosed herein are sequences incorporating a "Hy-"moiety at the amino terminus (N-terminus) of the sequence, and either an "-OH" moiety or an "-$NH_2$" moiety at the carboxy terminus (C-terminus) of the sequence. In such cases, and unless otherwise indicated, a "Hy-" moiety at the N-terminus of the sequence in question indicates a hydrogen atom [i.e. $R^1$ = hydrogen = Hy in the general formulas; corresponding to the presence of a free primary or secondary amino group at the N-terminus], while an "-OH" or an "-$NH_2$" moiety at the C-terminus of the sequence

indicates a hydroxy group [e.g. $R^2$ = OH in general formulas; corresponding to the presence of a carboxy (COOH) group at the C-terminus] or an amino group [e.g. $R^2$ = [$NH_2$] in the general formulas; corresponding to the presence of an amido ($CONH_2$) group at the C-terminus], respectively. In each sequence of the invention, a C-terminal "-OH" moiety may be substituted for a C-terminal "-$NH_2$" moiety, and vice-versa.

"Percent (%) amino acid sequence identity" with respect to the GLP-2 polypeptide sequences is defined as the percentage of amino acid residues in a candidate sequence that are identical to the amino acid residues in the wild-type (human) GLP-2 sequence, after aligning the sequences and introducing gaps, if necessary, to achieve the maximum percent sequence identity, and not considering any conservative substitutions as part of the sequence identity. Sequence alignment can be carried out by the skilled person using techniques well known in the art, for example using publicly available software such as BLAST, BLAST2 or Align software. For examples, see Altschul et al., Methods in Enzymology 266: 460-480 (1996) or Pearson et al., Genomics 46: 24-36, 1997.

The percentage sequence identities used herein in the context of the present invention may be determined using these programs with their default settings. More generally, the skilled worker can readily determine appropriate parameters for determining alignment, including any algorithms needed to achieve maximal alignment over the full length of the sequences being compared.

Each embodiment of the invention described herein may be taken alone or in combination with one or more other embodiments of the invention.

## Examples

### Preparation of Short Bowel Syndrome (SBS) mice

Adult, male C57BL/6J mice with ~ 30 g body weight of ~ 4 months received a 12-cm ileum and cecum resection (ICR) with jejunocolonic anastomosis (see figure 1, middle). The mice were then randomly assigned to either Cpd 18 or vehicle treatment As control for the abdominal surgery, the sham group was included which had laparotomy, bowel exposure, transection and end-to-end anastomosis but no resection (see figure 1, bottom). To prevent intestinal obstruction, all mice were switched to liquid diet 2 days before surgery and maintained on this diet until the end of the experiment. Throughout the experiment, animals were singly housed. All mice were studied with the intention to survive to day 14.

Briefly, mice were anesthetized by i. p. injection of ketamine (100 mg/kg bw) and xylazine (15 mg/kg bw) and orally intubated and ventilated. After midline incision, the distal small bowel and the cecum were exposed and then transected 12 cm proximal to the ileocecal junction and immediately distal to the cecum. The resected bowel was removed, and the length of the specimen was measured. An end-to-end, jejunocolonic anastomosis was created and the abdomen was closed. In the sham group, laparotomy, bowel exposure, transection and end-to-end anastomosis but no resection was performed. Immediately after surgery, all mice were weighed and received 1 ml isotonic saline subcutaneously and carprofen 5 mg/kg body weight. All surgical procedures were performed by the same surgeon. Mice were kept in a heated terrarium (29 °C) for 4 h and then returned to individual cages with free access to liquid food and water. The liquid diet was provided in feeding tubes. Mice were allowed to recover for a day after surgery, and then injected once daily with 30 nmol/kg body weight of Cpd 18. 50 mM Histidine buffer pH 6, 200 mM Mannitol served as vehicle control.

Mice were scored for wellbeing daily as described (Berlin et al. 2019). If the wellness score dropped to below 4 pts, or when weight loss exceeded 20%, the mouse was immediately killed and not analyzed further. On day 14 animals were animals were sacrificed by cervical dislocation and the small bowel excised for further study.

### Example 1: Observations on the mice used in the following examples

Sham and SBS mice were prepared as described under Preparation of Short Bowel Syndome (SBS) mice. The initial weight and initial age of the mice used are shown in figures 2 and 3. As seen from the figures there were no differences between the sham, vehicle or Cpd 18 (Compound 18) groups for the initial weight of the mice prior to surgery (figure 2), and no differences between the sham, vehicle or Cpd 18 groups for the initial age of the mice prior to surgery (Figure 3). There was an equal resection length between the vehicle or Cpd 18 groups, whilst there was no resection performed for the sham group, only an anastomosis (figure 4).

Figure 5 shows that vehicle treated mice had the lowest survival rate after 14 days (60%), whilst treatment with Cpd 18 gave an increased rate of survival (74%) as compared to vehicle treatment, sham operated animals had the highest survival rate (90%).

Body weight (figure 6) and food consumption (figure 7) was measured daily. The feeding tubes were weighed when replenished after 24h in the cage.

As seen from figure 6, all groups dropped to approximately 90% of the relative starting weight one day after surgery. The relative body weight of the sham group started to increase towards starting weight after just 2 days, whilst the

group treated with Cpd 18 differed from the vehicle group after six days onwards by starting to increase towards the initial weight, the vehicle group stayed relatively flat even after 14 days (Figure 6). The increase in relative body weight in Cpd 18 treated group as compared to the vehicle group was despite dietary intake (figure 7) being lower in the Cpd 18 treated animals from day 2 onwards as compared to vehicle.

**Example 2: Amount of water in stool**

Stools were collected on selected days throughout the experiment. Stool water content was assessed by collecting stools in a pre-weighed tube, weighing, and then drying overnight at 80°C and reweighing the dried stools. Figure 8 shows that before surgery all groups had stool water content of approximately 50%, the stool water content increased in the resected mice groups treated with both vehicle and Cpd18 after 2 days to approximately 75%, this was consistent with watery and yellow stools, whilst stool water content remained low in the sham group. Thereafter, stool water remained at 50% in the sham group and in the resected vehicle group at approximately 75% until day 14. However, in Cpd18 treated resected mice, stool water content was significantly reduced compared to vehicle from day 7 and even returned to levels of sham operated mice at approximately 50% on day 14.

**Example 3: Visual assessment of stool consistency**

Figure 9 shows examples of stools collected on different days of the SBS mouse experiment, from vehicle (V) treated or Cpd18 treated resected mice or sham treated animals. The stool consistency can be compared visually with reference to the Bristol Stool Assessment type usually used for assessment of human stools (figure 10).

The top panel of figure 9 shows stool from day 5 after resection, the two vehicle treated examples are of Type 6 or 7, whilst stool from the Cpd 18 treated animals could be classified as Type 4 or 5, whilst sham treated animals have stool of Type 3 or 4. The middle panel of figure 9 is from stools collected on day 10, again the stools from sham treated have a more watery and yellow consistency compared to those from sham or Cpd 18 treated animals. On day 14 the stool from the vehicle treated animals would still be classified as Type 6 or 7, whilst those from Cpd 18 treated animals are of type 4 similar to those from the sham treated animals.

<u>References</u>

Berlin P, et al. Villus Growth, Increased Intestinal Epithelial Sodium Selectivity, and Hyperaldosteronism Are Mechanisms of Adaptation in a Murine Model of Short Bowel Syndrome. Dig Dis Sci. 2019 May;64(5):1158-1170. doi: 10.1007/s10620-018-5420-x Reiner J, et al. Teduglutide Promotes Epithelial Tight Junction Pore Function in Murine Short Bowel Syndrome to Alleviate Intestinal Insufficiency. Dig. Dis. Sci. 2020 Feb 1.

Jeppesen, Teduglutide, a novel glucagon-like peptide 2 analog, in the treatment of patients with short bowel syndrome, Therapeutic Advances in Gastroenterology (2012) 5(3) 159-171

**Claims**

**1.** A composition comprising a GLP-1/GLP-2 dual agonist, which is Hy-H[Aib]EGSFTSELATILD[K([17-carboxy-heptadecanoyl]-isoGlu)]QAARDFIAWLIQHKITD-OH (Compound 18) or a pharmaceutically acceptable salt or solvate thereof, for use in a method for the treatment and/or prevention of diarrhea in a patient having undergone surgical resection of the bowel.

**2.** The composition for use in the method according to claim 1, wherein said patient has short bowel syndrome (SBS).

**3.** The composition for use in the method according to any of the preceding claims, wherein the patient has had a colostomy or an ileostomy.

**4.** The composition for use in the method according to any of the preceding claims, wherein the treatment and/or prevention reduces the frequency of daily defecations.

**5.** The composition for use in the method according to any of the preceding claims, wherein the treatment and/or prevention

(a) reduces the frequency of daily defecations by at least 30%, such as at least 40%, at least 50% or at least 60%; and/or

(b) reduces the number of daily defecations by at least one defecation.

6. The composition for use in the method according to any of the preceding claims, wherein the treatment and/or prevention reduces the amount of water in the feces of said patient by at least 20% compared to the amount of water in the feces of said patient before starting the treatment with said composition.

7. The composition for use in the method according to any of the preceding claims, wherein the treatment and/or prevention reduces the amount of water in the feces, allowing the Bristol Stool Score of the feces to be reduced by at least 1 on the Bristol Stool Scale.

8. The composition for use in the method according to any of the preceding claims, wherein the treatment and/or prevention improves the consistency of the feces.

9. The composition for use in the method according to any of the preceding claims, wherein the composition is administered to the patient for a period of at least 7 days.

10. The composition for use in the method according to any of the preceding claims, wherein said composition is a pharmaceutical composition.

11. The composition for use in the method according to any of the preceding claims, wherein said dual agonist is administered at a dose of about 0.1 pmol/kg to 500 μmol/kg body weight, preferably about 50 pmol/kg to 500 nmol/kg.

**Patentansprüche**

1. Zusammensetzung, umfassend einen GLP-1/GLP-2-Dualagonisten, bei dem es sich um Hy-H[Aib]EGSFTSELATILD[K([17-Carboxyheptadecanoyl]-isoGlu)]QAARDFIAWLIQHKITD-OH (Verbindung 18) oder ein pharmazeutisch akzeptables Salz oder Solvat davon handelt, zur Verwendung in einem Verfahren zur Behandlung und/oder Prävention von Diarrhöe in einem Patienten, der einer chirurgischen Resektion des Darms unterzogen wurde.

2. Zusammensetzung zur Verwendung in dem Verfahren nach Anspruch 1, wobei der Patient ein Kurzdarmsyndrom (SBS) aufweist.

3. Zusammensetzung zur Verwendung in dem Verfahren nach einem der vorhergehenden Ansprüche, wobei der Patient eine Kolostomie oder eine Ileostomie hatte.

4. Zusammensetzung zur Verwendung in dem Verfahren nach einem der vorhergehenden Ansprüche, wobei die Behandlung und/oder Prävention die Häufigkeit täglicher Defäkationen verringert.

5. Zusammensetzung zur Verwendung in dem Verfahren nach einem der vorhergehenden Ansprüche, wobei die Behandlung und/oder Prävention

(a) die Häufigkeit täglicher Defäkationen um mindestens 30 %, wie etwa mindestens 40 %, mindestens 50 % oder mindestens 60 % verringert; und/oder
(b) die Zahl der täglichen Defäkationen um mindestens eine Defäkation verringert.

6. Zusammensetzung zur Verwendung in dem Verfahren nach einem der vorhergehenden Ansprüche, wobei die Behandlung und/oder Prävention die Menge an Wasser in den Fäkalien des Patienten um mindestens 20 % im Vergleich zu der Menge an Wasser in den Fäkalien des Patienten vor Beginn der Behandlung mit der Zusammensetzung verringert.

7. Zusammensetzung zur Verwendung in dem Verfahren nach einem der vorhergehenden Ansprüche, wobei die Behandlung und/oder Prävention die Menge an Wasser in den Fäkalien verringert, wodurch ermöglicht wird, dass der Bristol-Stool-Score der Fäkalien um mindestens 1 auf der Bristol-Stool-Skala reduziert wird.

8. Zusammensetzung zur Verwendung in dem Verfahren nach einem der vorhergehenden Ansprüche, wobei die Behandlung und/oder Prävention die Konsistenz der Fäkalien verbessert.

9. Zusammensetzung zur Verwendung in dem Verfahren nach einem der vorhergehenden Ansprüche, wobei die Zusammensetzung dem Patienten für einen Zeitraum von mindestens 7 Tagen verabreicht wird.

10. Zusammensetzung zur Verwendung in dem Verfahren nach einem der vorhergehenden Ansprüche, wobei die Zusammensetzung eine pharmazeutische Zusammensetzung ist.

11. Zusammensetzung zur Verwendung in dem Verfahren nach einem der vorhergehenden Ansprüche, wobei der Dualagonist in einer Dosis von etwa 0,1 pmol/kg bis 500 μmol/kg Körpergewicht, vorzugsweise etwa 50 pmol/kg bis 500 nmol/kg, verabreicht wird.

## Revendications

1. Composition comprenant un agoniste double de GLP-1/GLP-2, qui est Hy-H[Aib]EGSFTSELATILD[K([17-carboxy-heptadecanoyl]-isoGlu)]QAARDFIAWLIQHKITD-OH (composé 18) ou un sel ou solvate pharmaceutiquement acceptable de celui-ci, pour utilisation dans un procédé de traitement et/ou de prévention de la diarrhée chez un patient ayant subi une résection chirurgicale de l'intestin.

2. Composition pour utilisation dans le procédé selon la revendication 1, dans laquelle ledit patient présente le syndrome du côlon court (SBS).

3. Composition pour utilisation dans le procédé selon l'une quelconque des revendications précédentes, dans laquelle le patient a subi une colostomie ou une iléostomie.

4. Composition pour utilisation dans le procédé selon l'une quelconque des revendications précédentes, dans laquelle le traitement et/ou la prévention réduisent la fréquence des défécations quotidiennes.

5. Composition pour utilisation dans le procédé selon l'une quelconque des revendications précédentes, dans laquelle le traitement et/ou la prévention

(a) réduisent la fréquence des défécations quotidiennes d'au moins 30 %, par exemple d'au moins 40 %, d'au moins 50 % ou d'au moins 60 % ; et/ou
b) réduisent le nombre de défécations quotidiennes d'au moins une défécation.

6. Composition pour utilisation dans le procédé selon l'une quelconque des revendications précédentes, dans laquelle le traitement et/ou la prévention réduisent la quantité d'eau dans les fèces dudit patient d'au moins 20 % par rapport à la quantité d'eau dans les fèces dudit patient avant de commencer le traitement par ladite composition.

7. Composition pour utilisation dans le procédé selon l'une quelconque des revendications précédentes, dans laquelle le traitement et/ou la prévention réduisent la quantité d'eau dans les fèces, permettant au score Bristol Stool des fèces d'être réduit d'au moins 1 sur l'échelle Bristol Stool.

8. Composition pour utilisation dans le procédé selon l'une quelconque des revendications précédentes, dans laquelle le traitement et/ou la prévention améliorent la consistance des fèces.

9. Composition pour utilisation dans le procédé selon l'une quelconque des revendications précédentes, dans laquelle la composition est administrée au patient pendant une période d'au moins 7 jours.

10. Composition pour utilisation dans le procédé selon l'une quelconque des revendications précédentes, dans laquelle ladite composition est une composition pharmaceutique.

11. Composition pour utilisation dans le procédé selon l'une quelconque des revendications précédentes, dans laquelle ledit agoniste double est administré à une dose d'environ 0,1 pmol/kg à 500 μmol/kg de poids corporel, de préférence d'environ 50 pmol/kg à 500 nmol/kg.

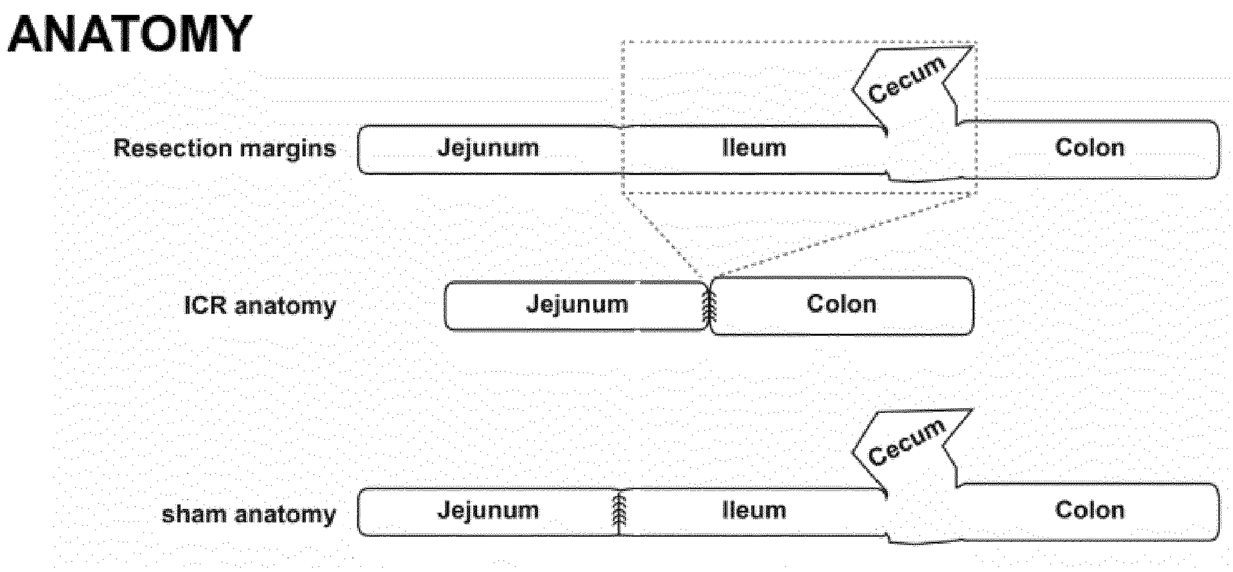


Figure 1

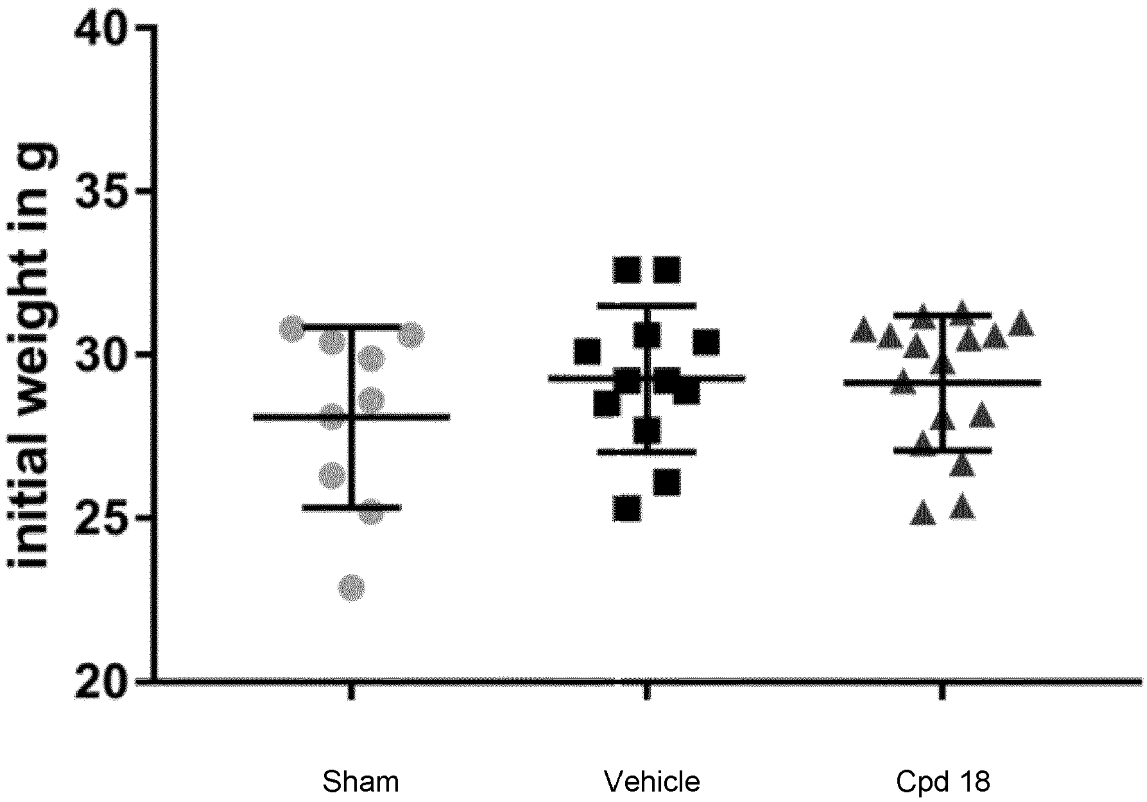


Figure 2

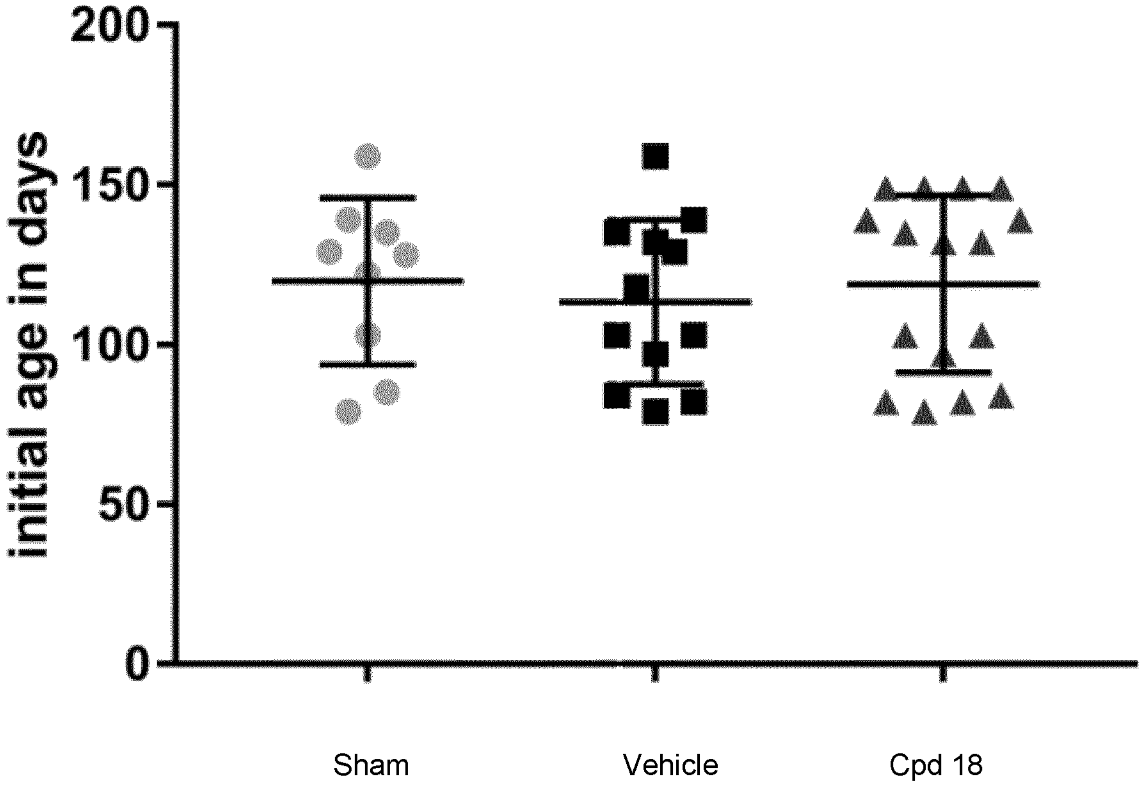


Figure 3

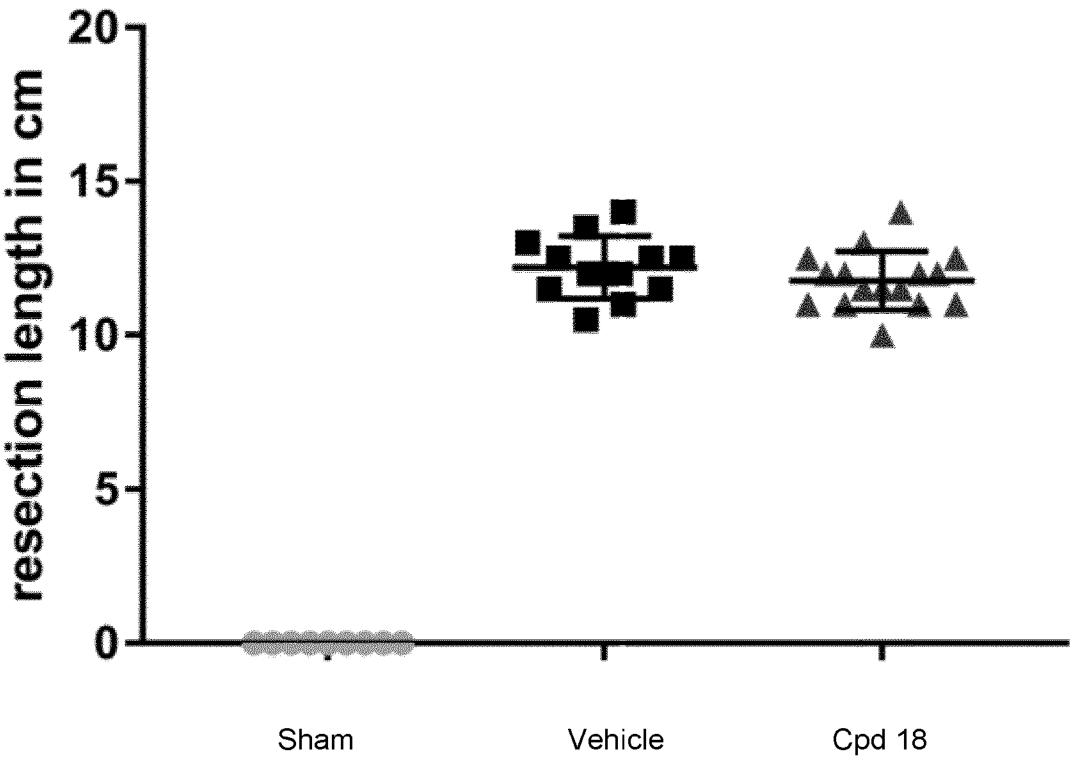


Figure 4

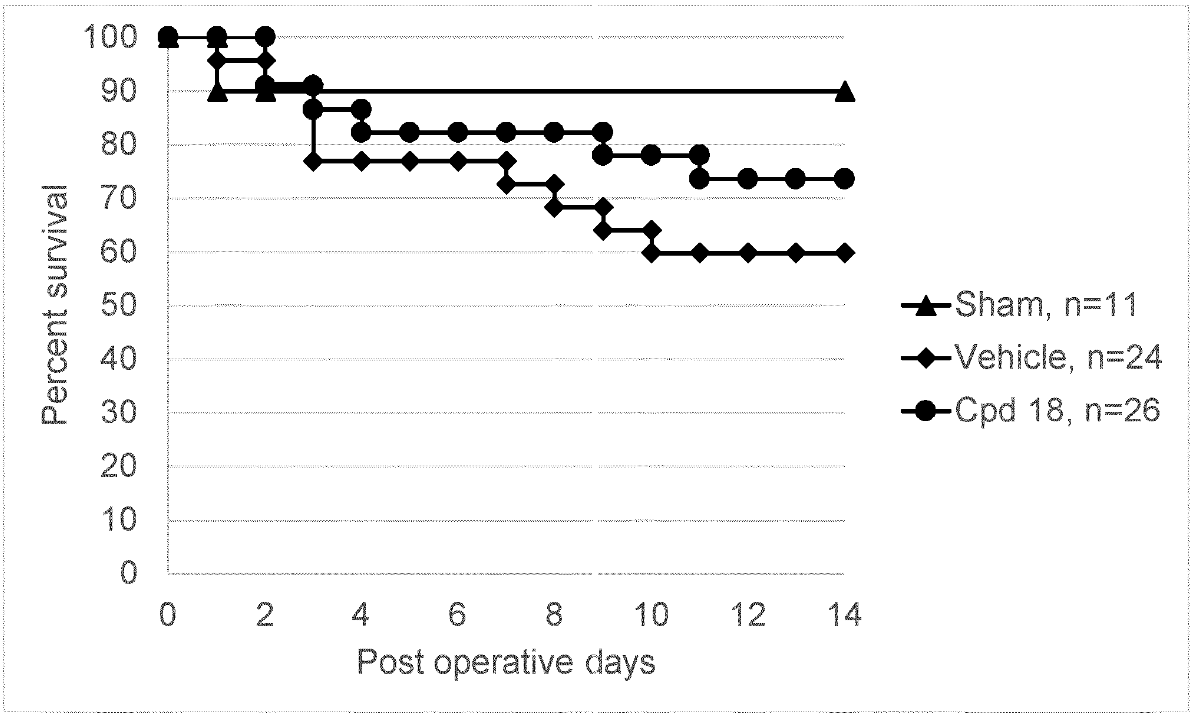


Figure 5

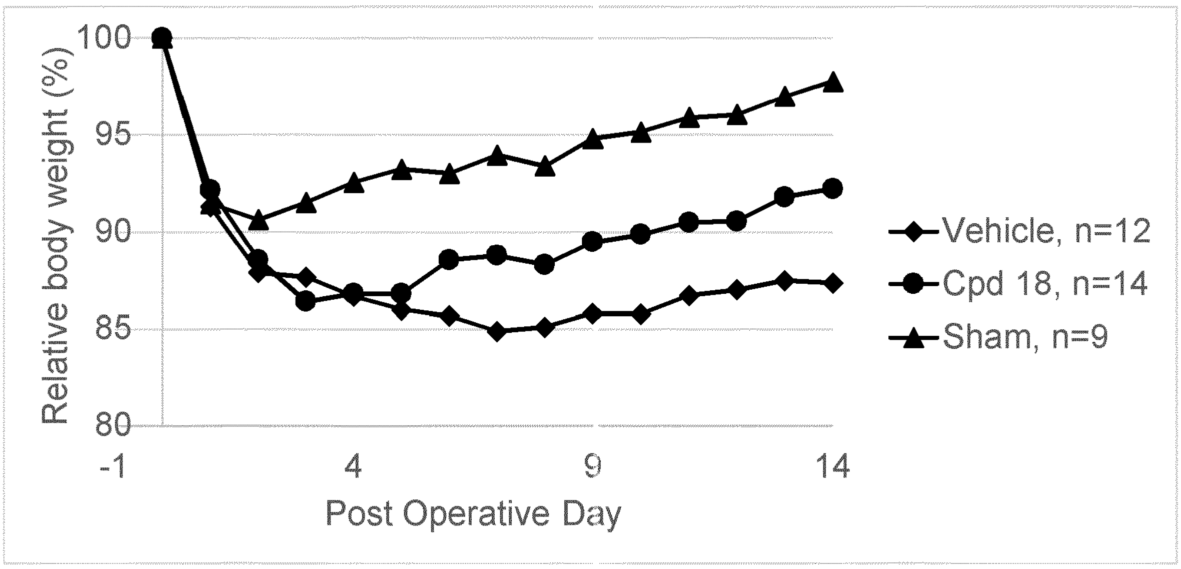


Figure 6

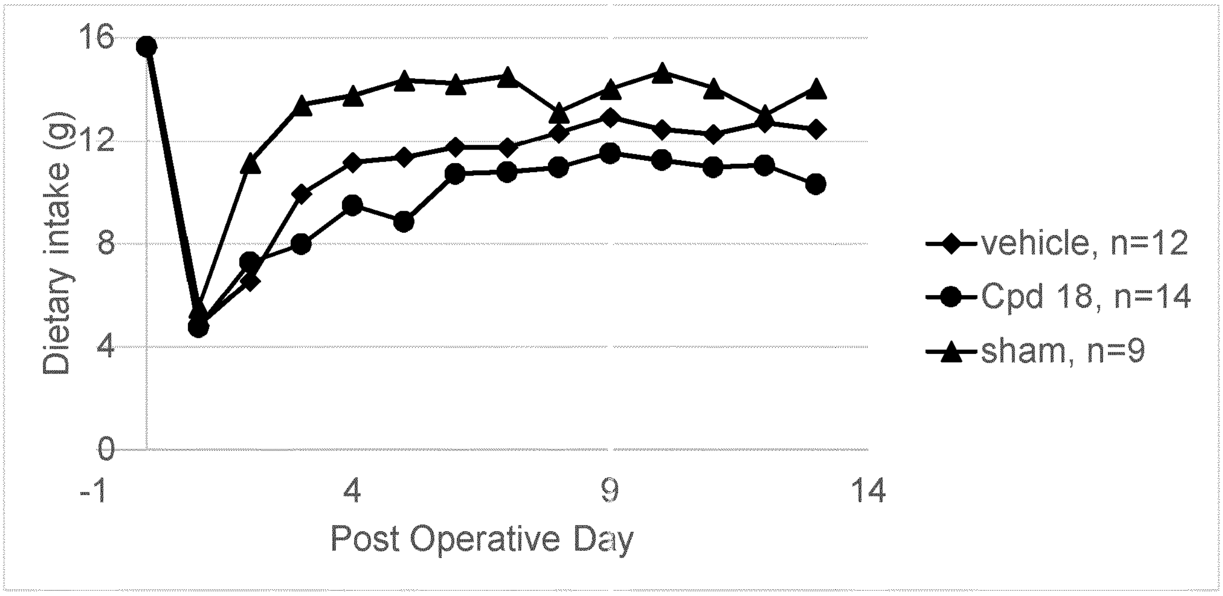


Figure 7

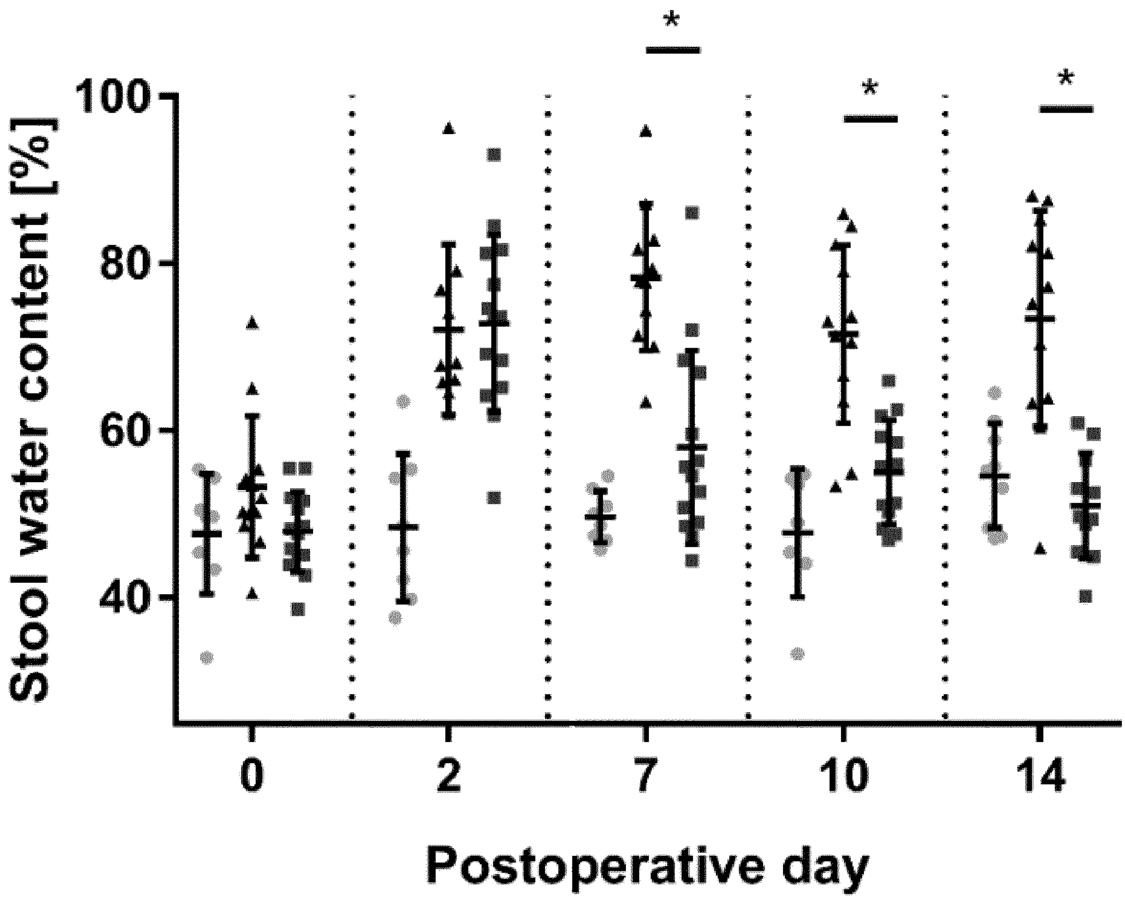


Figure 8

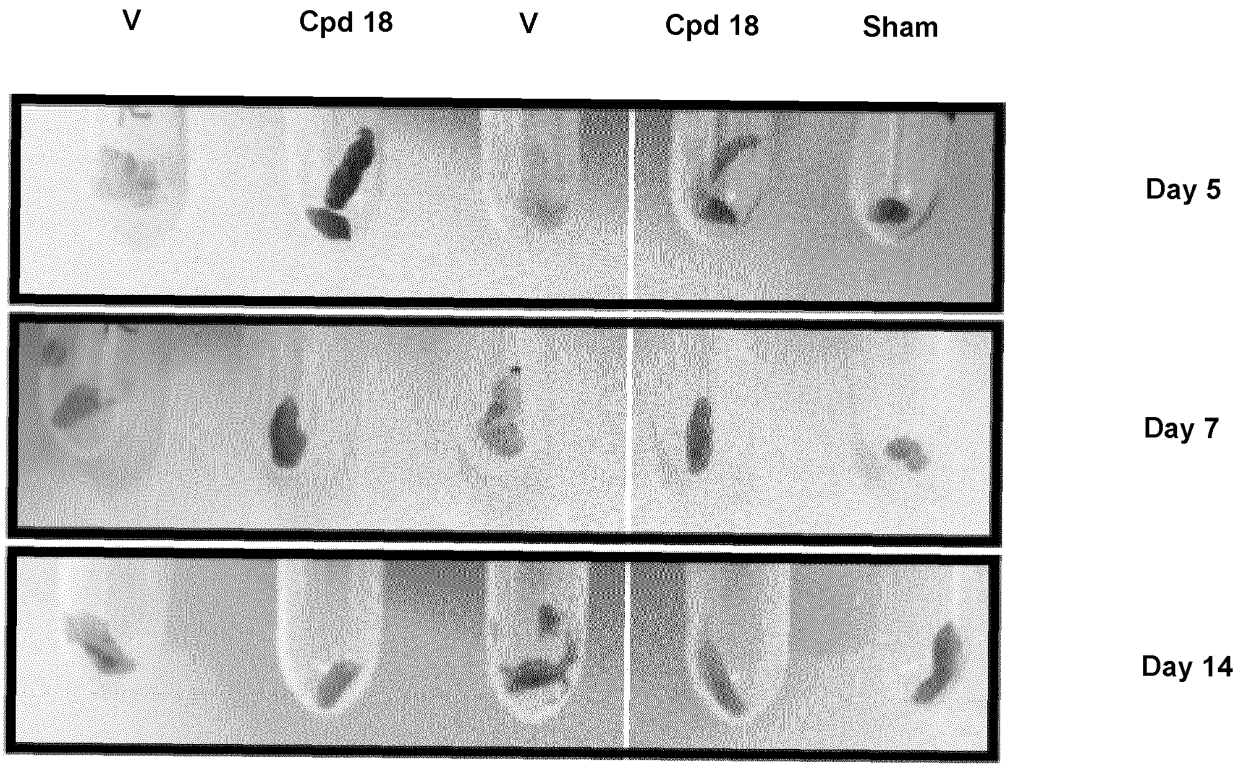


Figure 9

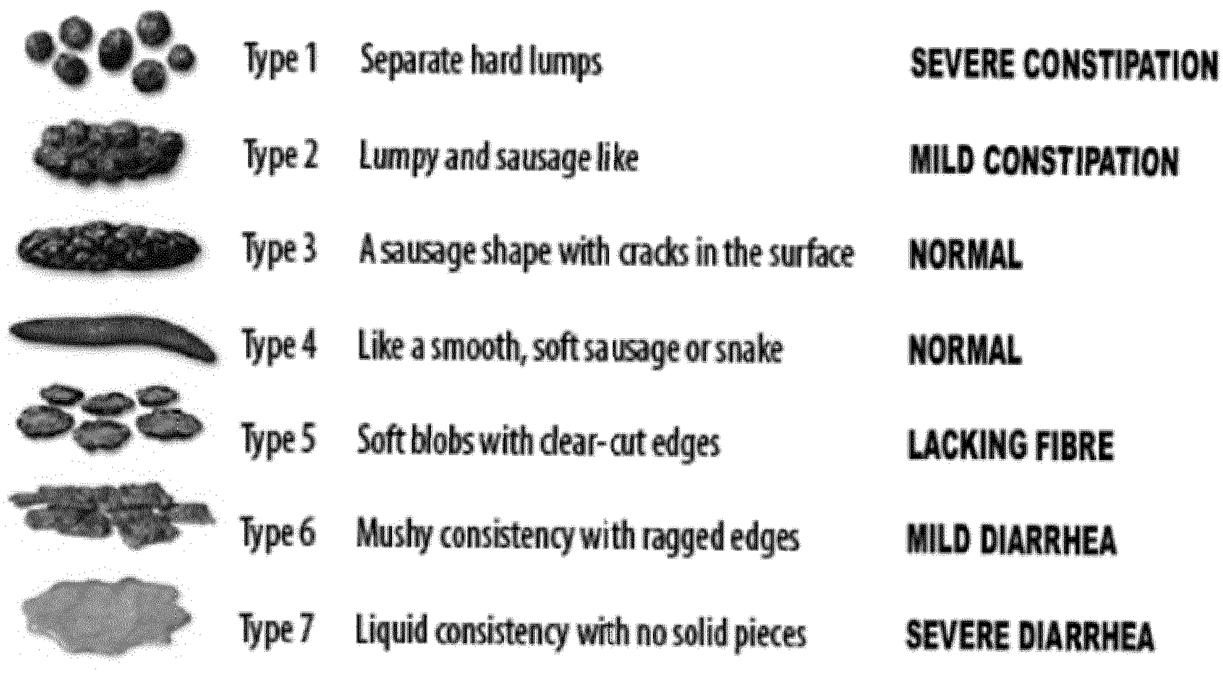


Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018104561 A1
- WO 2018104560 A1
- WO 2018104558 A1
- WO 2018103868 A1
- WO 2018104560 A
- WO 2018104561 A
- WO 9811125 A
- WO 2020249782 A1


### Non-patent literature cited in the description


- **J. SKARBALIENE et al.** PT01.2: ZP7570: A Novel GLP-1/GLP-2 Dual Agonist Peptide with Potential as the Next Generation Therapy for Short Bowel Syndrome. *CLINICAL NUTRITION*, 01 September 2019, vol. 38
- Principles and practice of solid-phase peptide synthesis. **FIELDS, G.B. et al.** Synthetic Peptides. 2002
- **ALTSCHUL et al.** *Methods in Enzymology*, 1996, vol. 266, 460-480
- **PEARSON et al.** *Genomics*, 1997, vol. 46, 24-36
- **BERLIN P et al.** Villus Growth, Increased Intestinal Epithelial Sodium Selectivity, and Hyperaldosteronism Are Mechanisms of Adaptation in a Murine Model of Short Bowel Syndrome.. *Dig Dis Sci.*, May 2019, vol. 64 (5), 1158-1170
- **REINER J et al.** Teduglutide Promotes Epithelial Tight Junction Pore Function in Murine Short Bowel Syndrome to Alleviate Intestinal Insufficiency.. *Dig. Dis. Sci.*, 01 February 2020
- **JEPPESEN**. Teduglutide, a novel glucagon-like peptide 2 analog, in the treatment of patients with short bowel syndrome. *Therapeutic Advances in Gastroenterology*, 2012, vol. 5 (3), 159-171